(19)
Europäisches Patentamt

European Patent Office

Office européen des brevets

(11) **EP 2 537 026 B1**

(12) **EUROPEAN PATENT SPECIFICATION**

(45) Date of publication and mention
of the grant of the patent:
**27.06.2018 Bulletin 2018/26**

(21) Application number: **11745323.3**

(22) Date of filing: **18.02.2011**

(51) Int Cl.:
*G01N 33/48* (2006.01)    *G01N 27/00* (2006.01)

(86) International application number:
**PCT/US2011/025434**

(87) International publication number:
**WO 2011/103424 (25.08.2011 Gazette 2011/34)**

(54) **HIGH-RESOLUTION ANALYSIS DEVICES AND RELATED METHODS**

HOCHAUFLÖSENDE ANALYSEVORRICHTUNGEN UND ZUGEHÖRIGE VERFAHREN

DISPOSITIFS D'ANALYSE À HAUTE RÉSOLUTION ET PROCÉDÉS APPARENTÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.02.2010 US 306114 P**

(43) Date of publication of application:
**26.12.2012 Bulletin 2012/52**

(73) Proprietor: **The Trustees of The University of
Pennsylvania
Philadelphia, PA 19104-6283 (US)**

(72) Inventors:
 • **DRNDIC, Marija
 Philadelphia, PA 19103 (US)**
 • **WANUNU, Meni
 Philadelphia, PA 19131 (US)**
 • **DADOSH, Tali
 Elkins Park, PA 19027 (US)**

(74) Representative: **Carpmaels & Ransford LLP
One Southampton Row
London WC1B 5HA (GB)**

(56) References cited:
**WO-A1-00/28312        WO-A2-2006/102292
WO-A2-2009/020682      US-A1- 2005 196 876**

**US-A1- 2005 196 876    US-A1- 2009 136 948
US-A1- 2009 142 825**

• WANUNU ET AL.: "Rapid electronic detection of
probe-specific microRNAs using thin nanopore
sensors.", NATURE NANOTECHNOL, vol. 5, no.
11, 24 October 2010 (2010-10-24), pages 807-814,
XP002699294,
• YOUNG-EUN CHOI ET AL: "Nanotechnology for
Early Cancer Detection", SENSORS, vol. 10, no.
1, 6 January 2010 (2010-01-06), pages 428-455,
XP055067238, DOI: 10.3390/s100100428
• NEELY ET AL.: "A single-molecule method for the
quantitation of microRNA gene expression.",
NAT METHODS, vol. 3, no. 1, January 2006
(2006-01), pages 41-46, XP008067118,
• NEELY ET AL.: 'A single-molecule method for the
quantitation of microRNA gene expression.' NAT
METHODS vol. 3, no. 1, January 2006, pages 41 -
46
• CHOI ET AL.: 'Nanotechnology for Early Cancer
Detection.' SENSORS vol. 10, no. 1, 06 January
2010, pages 428 - 455
• WANUNU ET AL.: 'Rapid electronic detection of
probe-specific microRNAs using thin nanopore
sensors.' NATURE NANOTECHNOL vol. 5, no. 11,
24 October 2010, pages 807 - 814
• M.?j. Kim ET AL: "Rapid Fabrication of Uniformly
Sized Nanopores and Nanopore Arrays for
Parallel DNA Analysis", Advanced Materials, vol.
18, no. 23, 4 December 2006 (2006-12-04), pages
3149-3153, XP055267464, DE ISSN: 0935-9648,
DOI: 10.1002/adma.200601191

EP 2 537 026 B1

**Description**

STATEMENT OF GOVERNMENT RIGHTS

This invention was made with government support under grant 1R21HG004767-01, awarded by the National Institutes of Health. The government has certain rights in the invention.

TECHNICAL FIELD

The present invention relates to the fields of solid state nanopores and of macromolecular analysis.

BACKGROUND

Nanopores in synthetic membranes hold great promise as platforms for next-generation DNA sequencing, as well as for other applications in genomics. Solid-state nanopores have been playing a major role for realizing these efforts, as they exhibit reproducible structure, scale-up capabilities, stability, and robustness. Low-stress silicon nitride (SiN), silicon oxide, and aluminum oxide have been used as membranes for the fabrication of solid-state nanopores.

Nanopore-based applications rely on reading the ion current of an electrolyte through the nanopore as biomolecules are threaded through the pore. The ion current highly depends on voltage, salt concentration, temperature, and the pore geometry. Analogous to the sharpness of an AFM tip, the length of the nanopore determines the overall resolution of the nanopore technique.

The reported thickness values of solid-state nanopores lie in the range of 20-50 nm, which provides a maximum readout resolution for double-stranded DNA of around 60-150 basepairs. This resolution, however, hinders the quality of information that is recovered from ion-current signals. Fabrication of thinner membranes, however, poses its own challenges, and is limited by physical stability, resulting in cracks and holes through the membrane that render the devices unusable. In light of the demand for a cheaper DNA sequencing, genomic analysis, RNA analysis, protein analysis, and other methods for ultrasensitive molecular analysis, there is a need in the art for ultrathin (e.g., < 10 nm) solid-state membrane substrates for nanopore analysis, and for related methods of fabricating and of using such devices.

SUMMARY

The present application provides approaches for fabricating sub-10 nm thick membrane devices, as well as the use of such membranes for nanopore-based nucleic acid analysis. This disclosure includes, inter alia, the steps of exposing a sub-micron region on a membrane (e.g., SiN) window, etching to locally thin the exposed region with sub-nanometer control, and the formation of a nanopore in the locally thinned region. Thereafter, the nanopore devices are treated using established protocols for subsequent biomolecular analysis.

In one aspect, the present invention provides analysis devices, the devices including a membrane having a thickness in the range of from about 0.2 to about 100 nm and comprising at least one pore extending therethrough, the pore having a characteristic cross-sectional in the range of from about 1 nm to about 1000 nm; and a supporting layer adjacent to the membrane.

In another aspect, provided are detection devices, the devices including a first capture material configured to bind preferentially to a first molecule; a membrane having a thickness in the range of from about 20 nm to about 100 nm, and the membrane having a thinned region, the thinned region having a thickness in the range of from about 0.1 nm to about 20 nm, and a first pore extending through the thinned region, the first pore being in fluid communication with the capture material; and a detector configured to detect a signal related to passage of the first molecule through the first pore.

The disclosure also provides methods of detecting an analyte, the methods including contacting a sample to a first capture material that preferentially binds to a first analyte; eluting the first analyte from the capture material; translocating the first analyte through a first pore disposed in a thinned region of a membrane, the thinned region having a thickness in the range of from about 0.1 nm to about 20 nm; and detecting a signal related to the translocation of the molecule through the first pore.

Also provided are methods of fabricating an analysis device, comprising removing at least a portion of a resist material disposed adjacent to a membrane so as to expose a target region of the membrane; etching at least a portion of the target region of the membrane material so as to reduce the thickness of the membrane material within the target region; and forming a pore that extends through the thinned target region of the membrane material.

Disclosed also are methods of fabricating an analysis device, the methods including removing at least a portion of a first material disposed adjacent to a membrane material having a thickness in the range of from about 20 nm to about 200 nm so as to expose at least one target region of the membrane material; and etching at least a portion of the at least one target region of the membrane material so as to reduce the thickness of the membrane material within the target region to from about 2 nm to about 30 nm.

The claimed invention also provides methods of analyzing a macromolecule, comprising translocating at least a portion of a macromolecule through a pore disposed in a membrane having a thickness in the range of from about 0.1 nm to about 20 nm; monitoring a signal related to the translocation of the macromolecule through the pore; and correlating the signal to a structural property of the macromolecule.

Additionally provided are membrane materials having a first region of from about 0.5 nm to about 20 nm in thickness; and a support layer disposed adjacent to the membrane material.

Further provided are analysis devices. These devices suitable include a membrane having a membrane thickness of from about 20 nm to about 1000 nm, the membrane having a thinned region thereon, and the thinned region having a thickness of between about 0.1 nm and about 20 nm.

Methods of analyzing a molecule are also provided. These methods include translocating at least a portion of a molecule through a pore disposed in a thinned region of a membrane, the thinned region having a thickness in the range of from about 0.1 nm to about 20 nm; and detecting a signal related to the translocation of the molecule through the pore.

The present application further provides detection devices. These devices suitably include a first capture material that binds specifically to a first molecule; a first membrane having a thickness in the range of from about 5 nm to about 100 nm; and a second membrane disposed adjacent the first membrane, the second membrane having a thickness in the range of from about 2 nm to about 20 nm, and the second membrane having at least one pore extending therethrough, the pore having a cross-sectional dimension in the range of from about 1 nm to about 100 nm, and the first membrane having a cavity formed thereon, the cavity being in register with at least one pore of the second membrane, the first pore being in fluid communication with the first capture material; a device configured to apply a gradient across the pore; and a detector configured to detect a signal related to passage of a molecule through the first pore.

Further included are methods of detecting an analyte. These methods include contacting a sample to a capture material that preferentially binds a first analyte; eluting the first analyte from the capture material; translocating the first analyte through a first pore formed in a first membrane disposed adjacent to a second membrane; the second membrane having a thickness in the range of from about 5 nm to about 100 nm and the second membrane having a cavity formed thereon, the cavity being in register with the first pore, the first membrane having a thickness in the range of from about 2 nm to about 20 nm, and the first pore extending through the first membrane, the pore having a cross-sectional dimension in the range of from about 1 nm to about 100 nm, the first pore being in fluid communication with the first capture material; and detecting a signal related to the translocation of the molecule through the pore.

Also provided are methods of fabricating a microscopy support. These methods include removing at least a portion of a first material disposed adjacent to a membrane so as to expose a first region of the membrane, the exposed region of the membrane having a thickness in the range of from 0.1 nm to about 20 nm.

BRIEF DESCRIPTION OF THE DRAWINGS

The summary, as well as the following detailed description, is further understood when read in conjunction with the appended drawings. For the purpose of illustrating the invention, there are shown in the drawings exemplary embodiments of the invention; however, the invention is not limited to the specific methods, compositions, and devices disclosed. In addition, the drawings are not necessarily drawn to scale. In the drawings:

Figure 1 depicts a completed device according to the claimed invention;
Figure 2 depicts a top-down view of a device according to the claimed invention;
Figure 3 depicts a device according to the claimed invention in an intermediate, nanopore-free stage of fabrication;
Figure 4 depicts a device according to the claimed invention in an intermediate, nanopore-free stage of fabrication where resist material (6) is present atop the capping layer (4);
Figure 5 depicts a device according to the claimed invention in an intermediate, nanopore-free stage of fabrication where resist material (6) is removed;
Figure 6 depicts a device according to the claimed invention in an intermediate, nanopore-free stage of fabrication where capping material (4) is removed;
Figure 7 depicts a device according to the claimed invention in an intermediate, nanopore-free stage of fabrication where resist material present in Figure 6 has been removed;
Figure 8 depicts a device according to the claimed invention in an intermediate, nanopore-free stage of fabrication where nanopore drilling has been initiated;
Figure 9 depicts a device according to the claimed invention where a nanopore has been formed in the membrane (3);

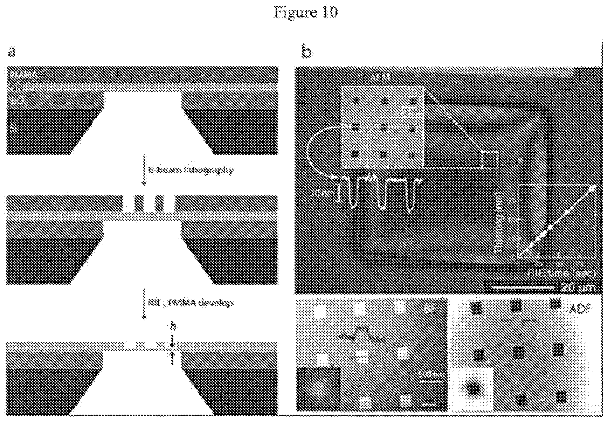

Figure 10 illustrates (a) a process for local thinning of solid-state membranes for improving the nanopore resolution, (b) optical microscope image of a pattern of squares used for thinning experiments. Following RIE process and lift-off, AFM image of the 250 nm squares is shown in the inset (17 nm thinning). The thinning was checked for different RIE times and shows excellent linearity (slope: 1 nm/sec). The bottom shows bright-field (BF) and annular dark field (ADF) images of the etched membranes, as well as corresponding images for a 10 nm pore (inset). The intensity profiles shown adjacent to the square-shaped pores highlight the exceptional contrast of ADF imaging, useful for accurate thickness determination.

**Figure 11** illustrates the detection of 25 bp dsDNA using a 3 nm solid-state nanopore according to the claimed invention in a 20 nm thick SiN membrane (T = 0° C., V = 300 mV). Concatenated single-molecule traces are shown. The small pore and low temperature combination facilitates detection of these short molecules (mean transport time = 80 microseconds), an important step for detecting RNA-drug complexes in design-RNA sequences, such as the ribosomal A-site and the TAR site of HIV RNA;

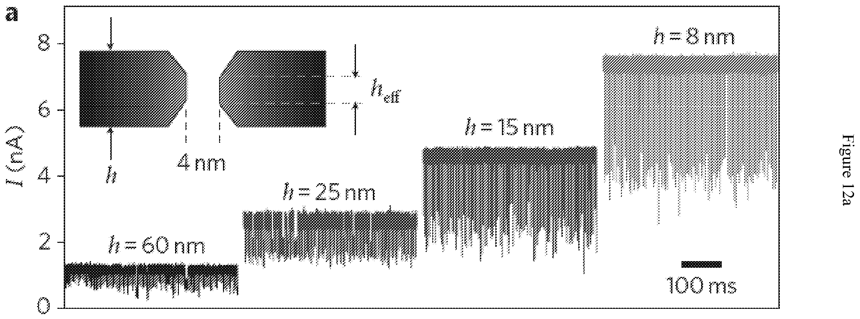

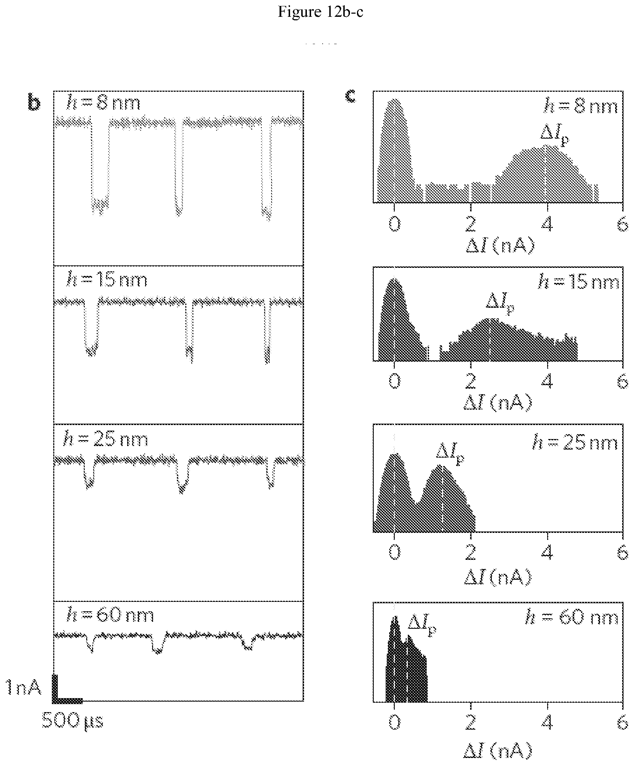

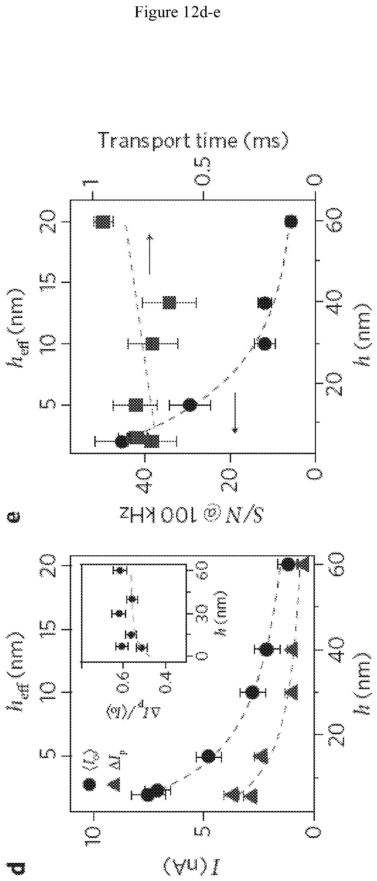

**Figure 12** illustrates Increasing the measurement resolution by nanopore thinning. (a) Concatenated sets of ~200 translocations of 3 kb linear double-stranded DNA through 4 nm diameter pores fabricated in membranes with different $h$ values; $h_{eff}$ is the nanopore's effective thickness used in the geometric model discussed in the text. In decreasing $h$ from 60 nm to 6 nm, the open pore current increased and the DNA signal amplitude increased. All traces were filtered using the Axopatch 100 kHz filter setting. For $h$ = 60 nm, the data was low-pass filtered at 10 kHz using the Axopatch filter to make events visible. (b) A magnified view of the traces in (a). (c) Semi-log histograms of the blocked current amplitudes normalized by subtracting *delta I*, which show increased current amplitudes for thinner nanopores. While the most probable blocked current $\Delta I_p$ increased with decreasing $h$, open pore noise values were similar. (d) Dependence of average experimental $<I_o>$ (circles) and the most probable DNA current amplitude $\Delta I_p$ (triangles) on $h$. The dashed line is a fit using Eqn. 1 to the average $I_o$ data from combined data of ~20 pores, which yields an effective pore thickness $h_{eff}$ = $h/(3.04 \pm 0.30)$ ($h_{eff}$ scale shown on top x-axis). The fit to $\Delta I_p$ values (dashed line) is based on a geometric model described in detail herein. The inset shows $\Delta I_p/<I_o>$, which did not change appreciably with $h$. The inset's dashed line is the ratio of the fits to $\Delta I_p$ and $<I_o>$ from the main plot. (e) The signal-to-noise (S/N) and mean transport time as a function of $h$ ($h_{eff}$ shown on top x-axis). One may define $S/N$ = $\Delta I/I_{RMS}$, where $I_{RMs}$ at 100 kHz bandwidth is $75 \pm 5$ pA. Mean transport times were obtained from the dwell-time distributions (see Ref 46 for more details).

**Figure 13** depicts an embodiment of the claimed invention wherein a nanopore has been formed in a locally thinned region of the membrane material (3);

**Figure 14** depicts an intermediate stage of nanopore device fabrication where a resist (6) is disposed atop a capping layer (4) and a membrane material (3). A dielectric layer (2), support layer (1), and additional support (0) are also present;

**Figure 15** illustrates electron beam removal of the resist material;

**Figure 16** illustrates removal of the capping layer (4) and membrane (3) so as to "thin" a region of the membrane layer (3);

**Figure 17** illustrates the formation ("drilling") of a nanopore in the thinned membrane region of Figure 16;

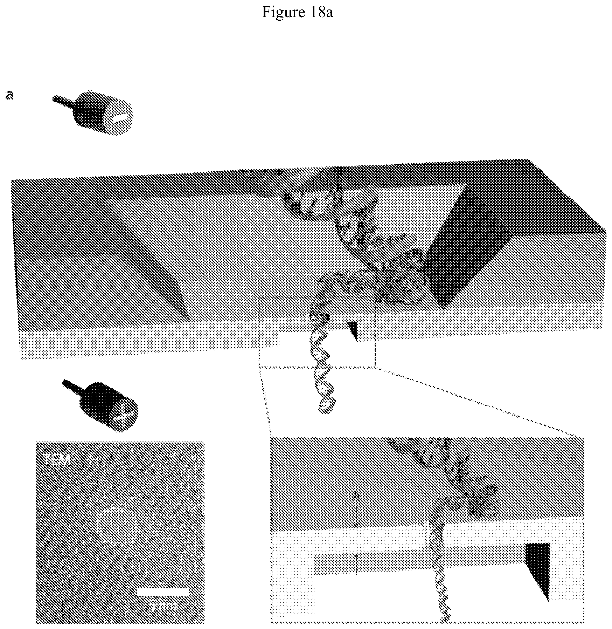

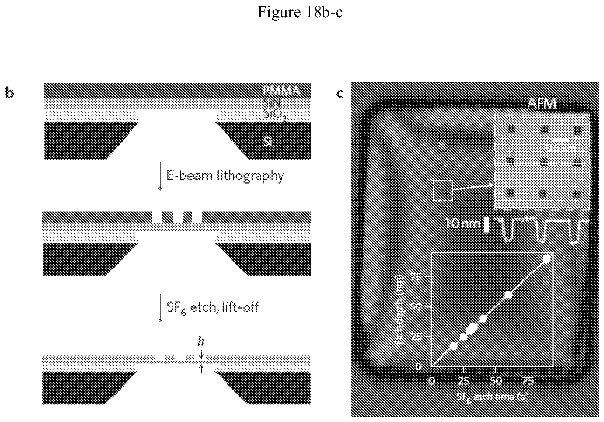

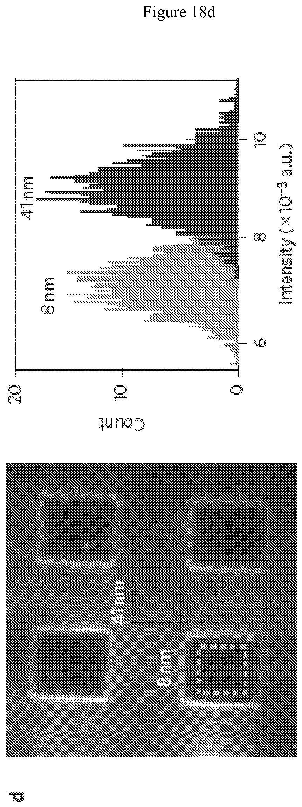

**Figure 18** illustrates sub-10 nm thick solid-state nanopore sensors. (a) Scheme of a nanopore sensor showing a DNA molecule translocating through the pore (not to scale). The sensor consists of a 5x5 mm2 Si chip that contains a freestanding silicon nitride (SiN) membrane (~50x50 $\mu$m$^2$). After locally thinning the membrane using the process shown in (b), a nanopore is drilled using a TEM (see TEM image of a 4 nm diameter nanopore in 6 nm thick membrane). Electrolyte solution is added above and below the nanopore, each contacted by a Ag/AgCl electrode, and voltage is applied to drive charged biomolecules through the pore. (b) The membrane thinning process involves coating the membrane with a PMMA resist, followed by e-beam exposure and development, and controlled dry etching using SF6 plasma. (c) Optical image of the membrane after thinning (before removal of the PMMA). The inset shows an AFM topography image of a 3x3 square array following PMMA removal, as well as a line profile that shows uniform, 17 nm deep trenches. The inset shows that the etch depth, measured by AFM, is a linear function of the etch time and that the etch rate is 1 nm/s. (d) Epi-fluorescence image of a 41 nm thick SiN membrane in which 5 $\mu$m squares were thinned to 8 nm ($\lambda$ex = 488 nm, $\lambda$em = $525 \pm 25$ nm). The fluorescence intensity histograms show lower fluorescence background in the thinned region;

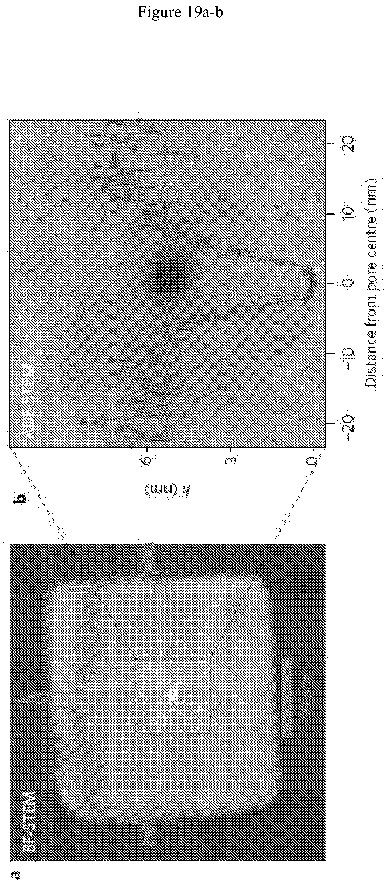

**Figure 19** illustrates scanning TEM (STEM) characterization of a 4.5 nm diameter pore in a 7 nm thick membrane (STEM probe size = 0.2 nm). (Left) A bright-field STEM (BF-STEM) image shows the etched 250 x 250 nm square as a brighter area with uniform intensity. (Right) Annular dark-field STEM (ADF-STEM) of a zoomed-in portion of the nanopore on the left. The height profile (data trace line on Figure 19a) of a line through the center of the pore is shown. The membrane thickness $h$ (y-axis) was measured from the difference of the initial membrane thickness and the etch depth (see text), normalized by assigning a thickness of 0 nm to the signal intensity at the pore (i.e., in vacuum).

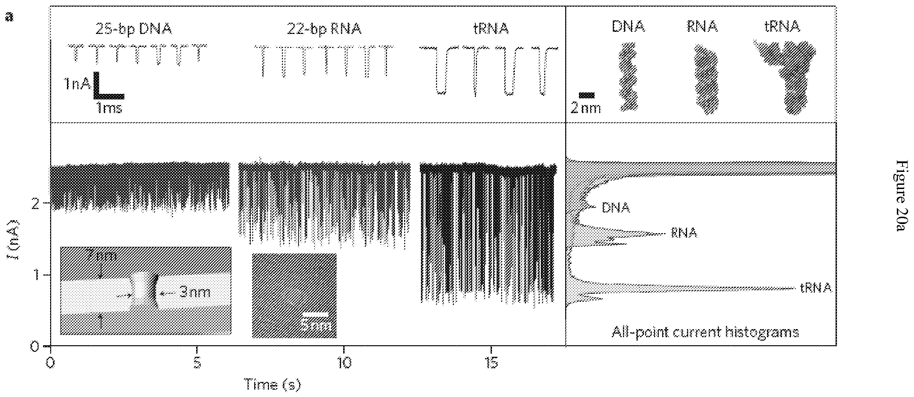

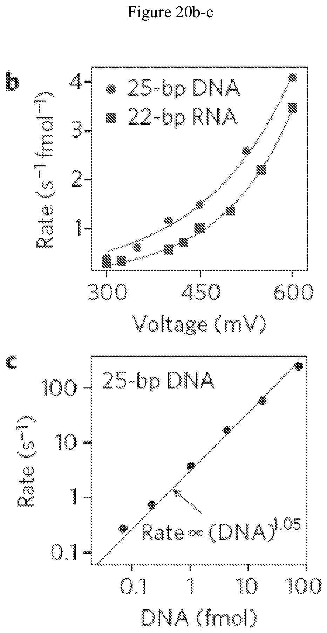

**Figure 20** illustrates discrimination among small nucleic acids using thin nanopores. (a) Continuous current vs. time traces from a 3 nm diameter pore in a 7 thick membrane measured at 0°C, V = 500 mV (TEM image of pore is shown). Traces were median-filtered with rank of 1 in order to improve the signal-to-noise. Based on the conductance, the effective pore thickness heff = 2.3 nm. The analyte chamber contains 25-bp dsDNA (left), 22-bp dsRNA (middle), or phenylalanine tRNA (right), at concentrations of ∼80 fmol/μl. Sample events are shown above the continuous traces, and models based on crystal structures are shown to the right

The all-point current histograms on the bottom right of Figure 20a show that molecules can be distinguished based on their current amplitudes. The mean transport times for the DNA, RNA, and tRNA molecules are 20 μs, 50 μs, and 1.04 ms, respectively. Part (b) of this figure shows the relationship of the capture rate on applied voltage for 25-bp DNA and 22-bp RNA. The exponential dependence reveals that capture is voltage-activated. The lines in Figure 20b are exponential fits to the data. Part (c) of the figure is a log-log plot of capture rate vs. DNA concentration. Linearity is observed for three orders of magnitude in DNA concentration, as indicated by a power-law fit exponent of 1.05 +/- 0.03;

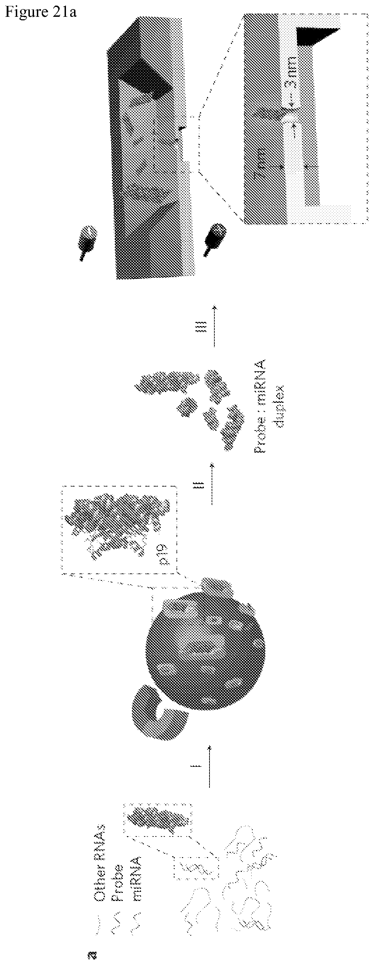

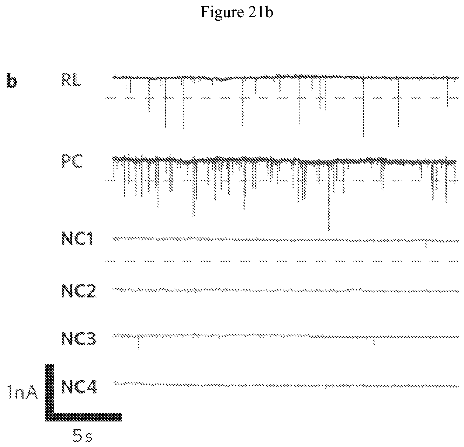

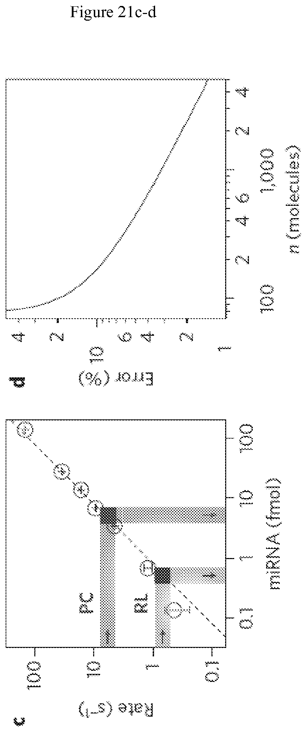

**Figure 21** illustrates microRNA detection using solid-state molecular counters. Detection (of miRNA or of other analytes) can include counting events (e.g., analyte passages) at the nanopore. The counted events can be correlated to a property of the source of the analyte. For example, the user may construct a calibration curve that allows them to relate the number of passage events to the concentration of an analyte in a sample.

Figure 21a illustrates a scheme for a miRNA-specific detection method. First, RNA is extracted from tissue (not shown; can be accomplished by lysing), and the extract is hybridized to a miRNA-specific oligonucleotide probe. In step (I), the probe:miRNA duplex is enriched by binding to p19-functionalized magnetic beads, followed by thorough washing in order to remove other RNAs from the mixture. In step (II) of Figure 21a, the hybridized probe:miRNA duplex is eluted from the magnetic beads. While magnetic beads are illustrated here, other support materials - e.g., porous supports, strips, and the like - may also be used. In step (III), the eluted probe:miRNA duplex is electronically detected using a nanopore. Figure 21b illustrates the detection of miR122a from rat liver RNA using a 3 nm diameter nanopore in a 7 nm thick membrane. The method shown in (a) was applied to detect miR122a from 1 μg of rat liver total RNA. Representative 30-second current vs. time traces are shown for a pore after the addition of the enriched miR122a (RL), a positive control containing a synthetic miR122a RNA duplex bound to magnetic beads, followed by washing, elution, and detection (PC), and four different negative controls (NC1-NC4, as described elsewhere herein). The negative controls did not produce any signal below the threshold, which was set to Io-0.4nA (see dashed lines in Fig. 21b). Figure 21c represents the quantification of miR122a from the mean capture rates. A calibration curve of capture rate vs. concentration was constructed (dashed line) using different concentrations of synthetic 22-bp RNA duplex, showing that capture rate scales linearly with concentration over three orders of magnitude. Determination of miR122a amounts (per μl solution) is based on the spike rate for sample RL ("RL" lines) and the positive control PC ("PC" lines). Figure 21d illustrates the relative error in the determined RNA concentration as a function of the number of molecules counted by the nanopore . To achieve 95% accuracy under exemplary conditions, the time required for determination of 1 fmol RNA sample is 4 minutes, corresponding to ∼250 translocation events;

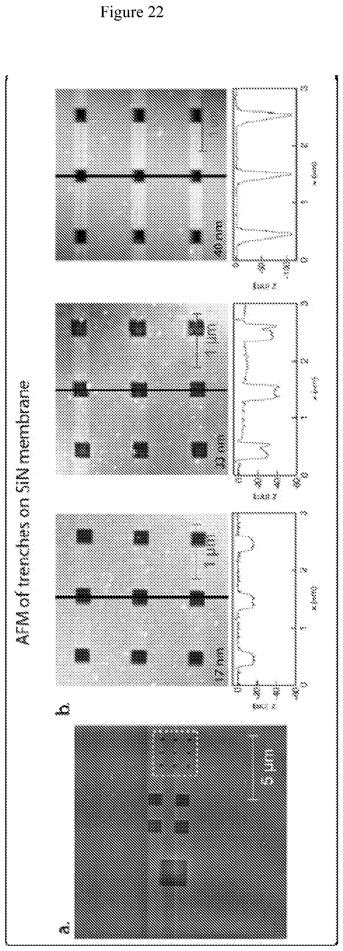

**Figure 22** illustrates AFM images of the pattern shown in figure 18;

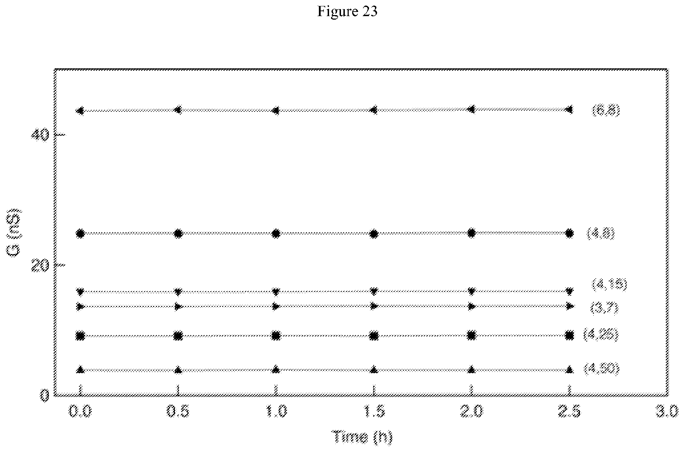

**Figure 23** illustrates the conductance as a function of time for pores with various diameters d and membrane thicknesses h, denoted as (d, h);

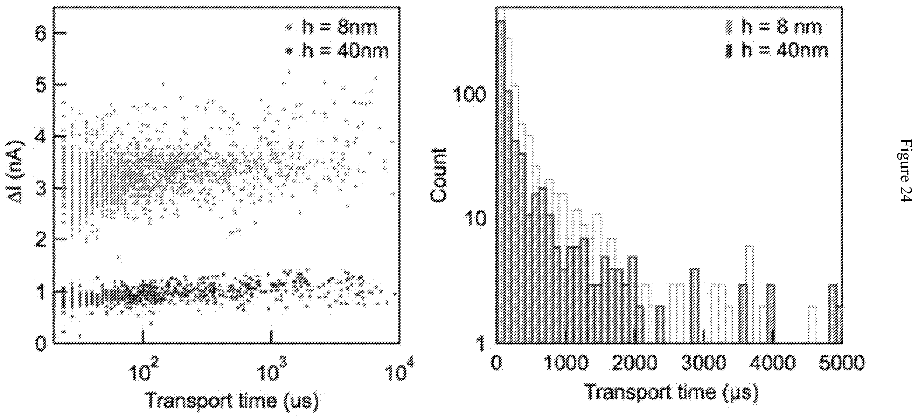

**Figure 24** illustrates a scatter plot of the mean current amplitude of each molecule (□I) and the total transport time for 3 kbp dsDNA through 4 nm pores as a function of membrane thickness (h);

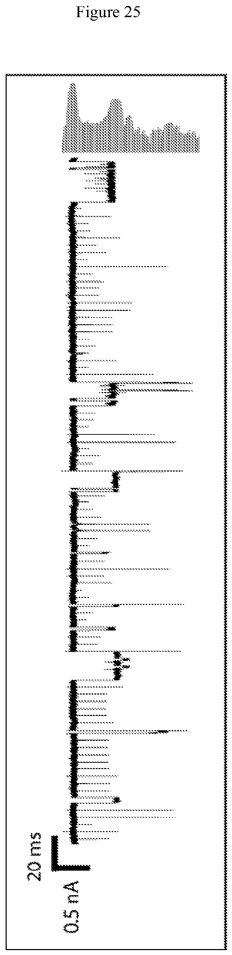

**Figure 25** illustrates a set of 10 bp translocations through a 3 nm diameter pore in a 7 nm thick membrane under 500 mV applied voltage, at a temperature of 0°C;

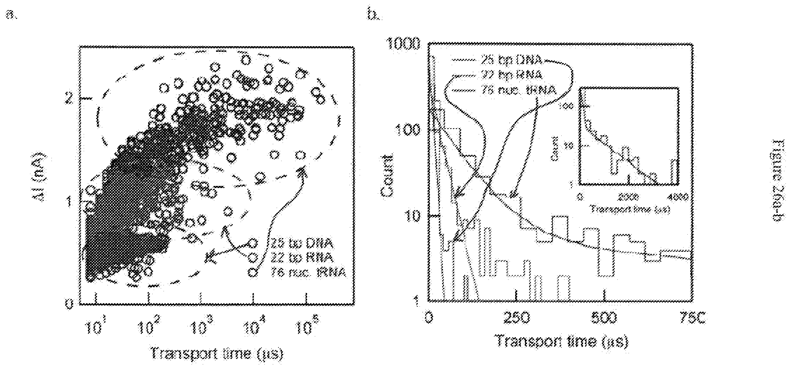

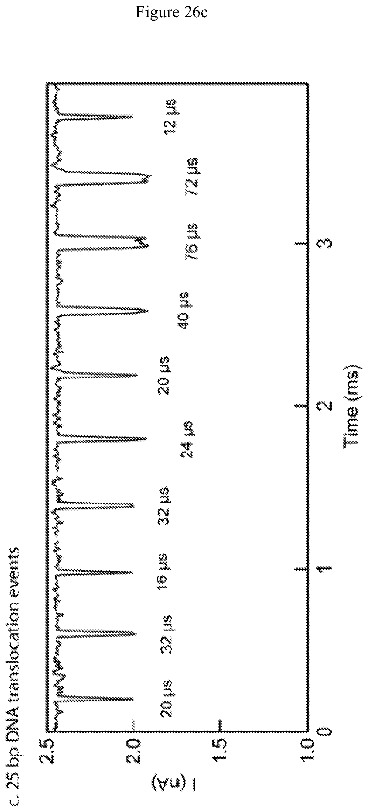

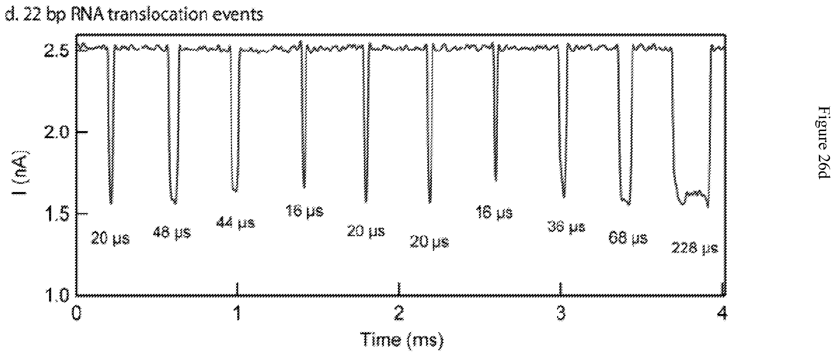

**Figure 26** illustrates using a 3 nm diameter pore in a 7 nm thick membrane to discriminate among small nucleic acids of similar size, namely, 25 bp DNA (molecular weight = 15kD), 22 bp RNA (molecular weight = 15 kD), and 76-nucleotide tRNA (molecular weight = 25 kD);

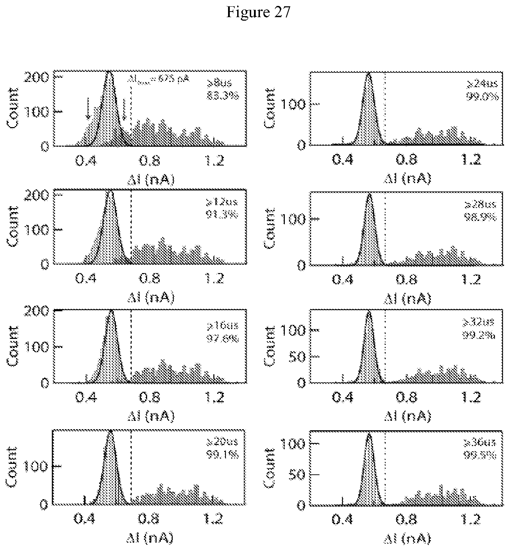

**Figure** 27 illustrates a quantitative analysis of a nanopore's ability to discriminate 25-bp DNA from 22-bp RNA based on current amplitudes;

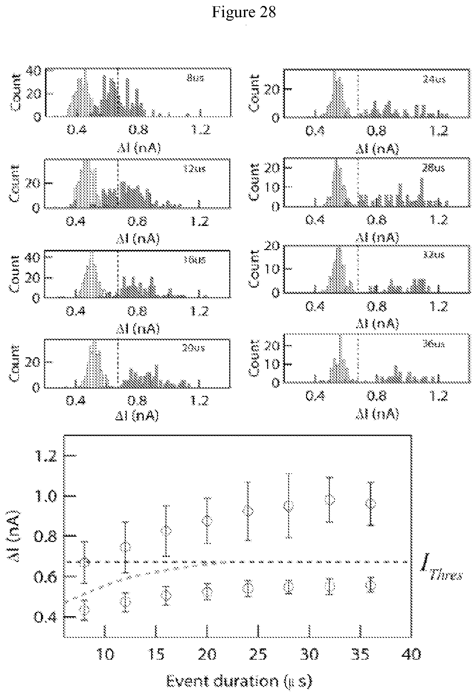

**Figure 28** illustrates page □I histograms for DNA and RNA events where in each plot is analyzed slices of the data that select all events with an indicated duration, in the range of 8-36 microseconds;

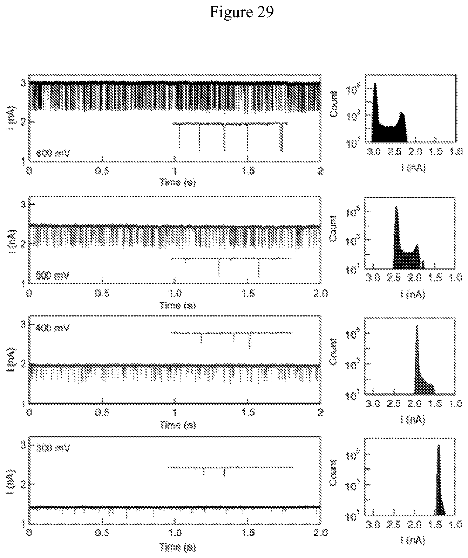

**Figure 29** illustrates shows 2-second current traces under different applied voltages of a 25 bp DNA sample analyzed using a 3 nm diameter pore fabricated in a 7 nm thick membrane (data taken at a temperature of 0°C);

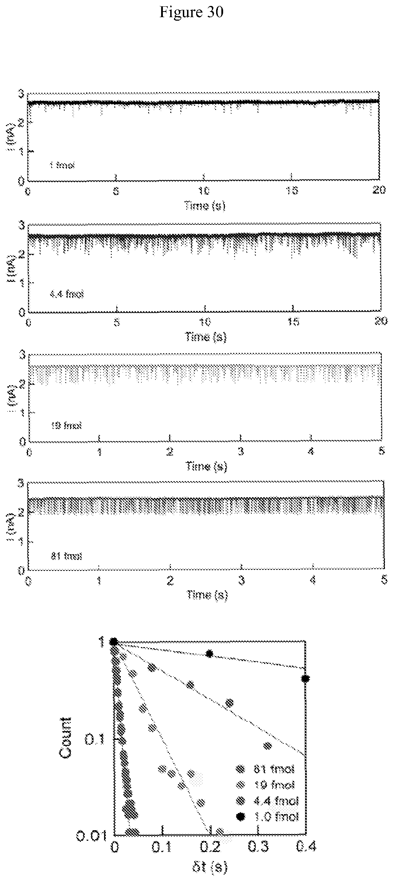

**Figure 30** illustrates current vs. time traces for a 3 nm diameter pore at a measured at a voltage of 500 mV and a temperature of 0°C, when different concentrations of DNA were added to the pore (expressed as fmol/μl solution);

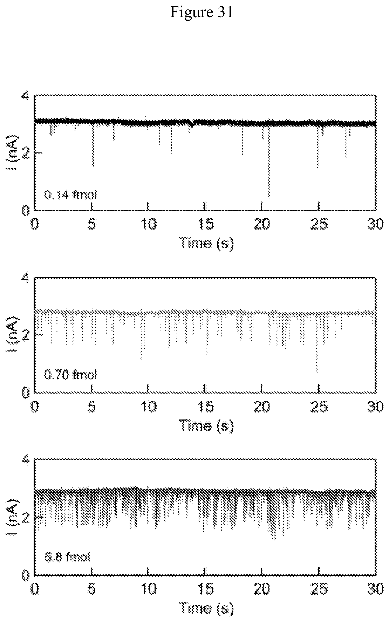

**Figure 31** illustrates continuous time traces for a 3 nm diameter pore measured at a voltage of 500 mV and a temperature of 0°C, when different concentrations of RNA were added to the pore (expressed as fmol/μl solution);

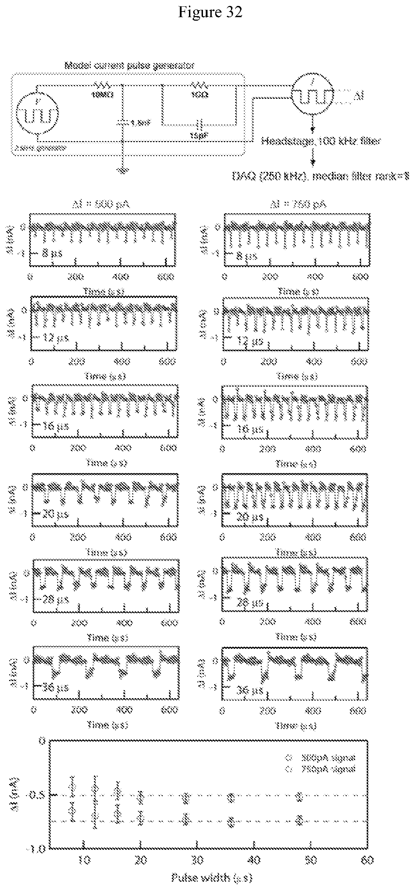

**Figure 32** illustrates the response of an exemplary, non-limiting amplifier to synthetic current pulses in the range 8 - 48 □s;

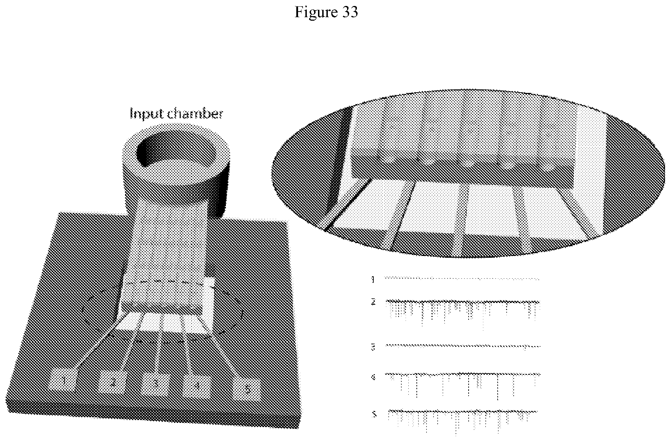

**Figure 33** illustrates an exemplary, non-limiting device analyte (e.g., miRNA) isolation and detection;

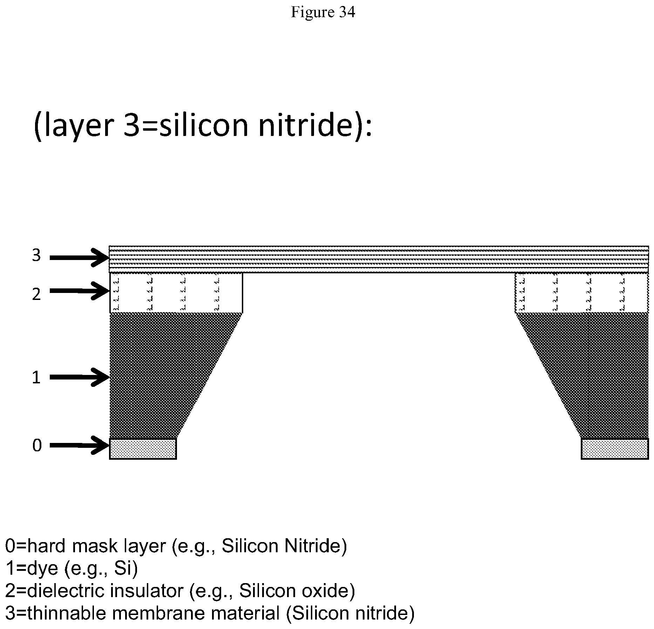

**Figure 34** illustrates a plan view of an exemplary device before processing, cross-section view;

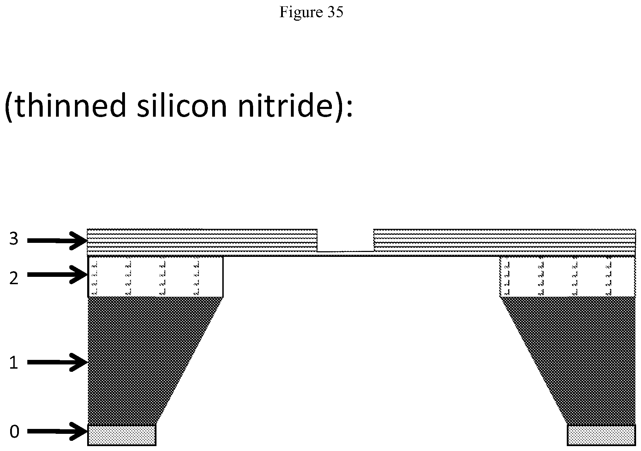

**Figure 35** illustrates plan view of the device after processing/thinning the thin membrane;

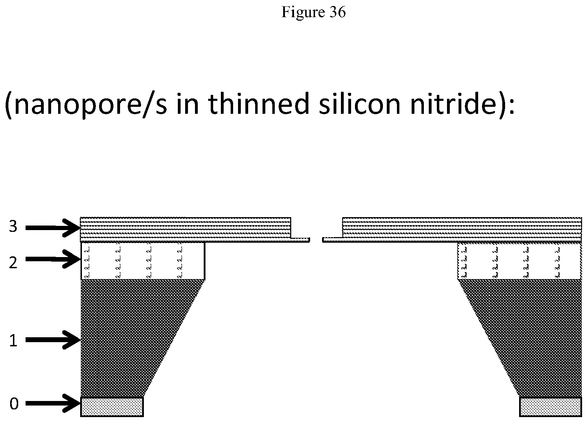

**Figure 36** illustrates an exemplary device after processing a pore in a thinned membrane;

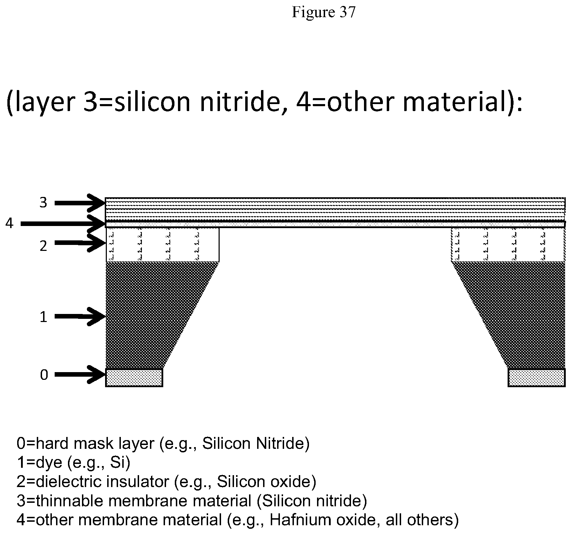

**Figure 37** illustrates an exemplary device;

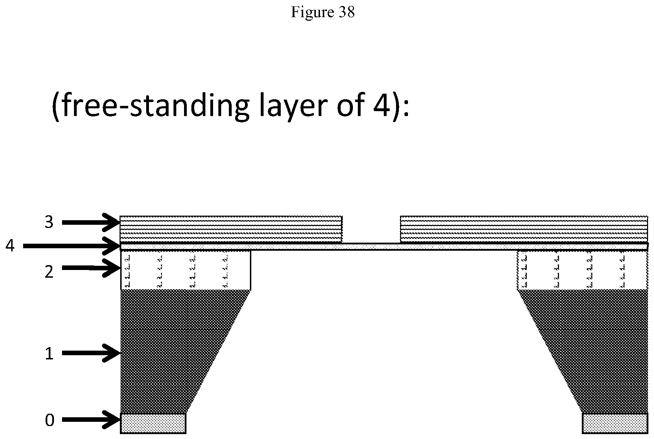

**Figure 38** illustrates an exemplary device having a cavity formed above a thin membrane;

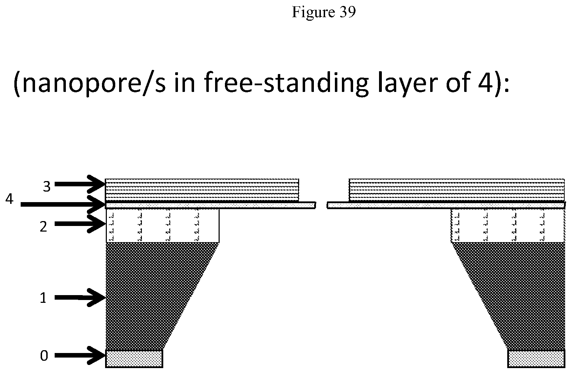

**Figure 39** illustrates the device of Figure 39 having a nanopore formed in the thin membrane; and

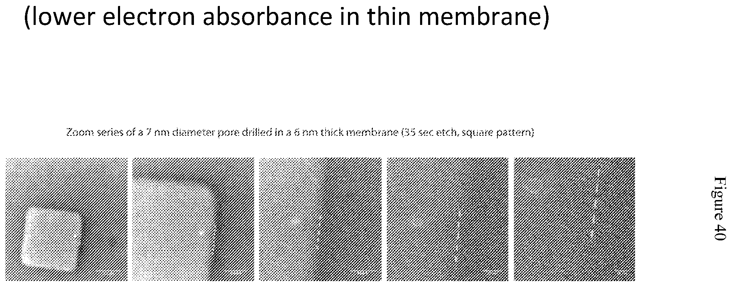

**Figure 40** illustrates images of a thin membrane region (light-color region; useful as a sample support in microscopy applications) and a thicker support material (darker color region).

DETAILED DESCRIPTION OF ILLUSTRATIVE EMBODIMENTS

The present invention may be understood more readily by reference to the following detailed description taken in connection with the accompanying figures and examples, which form a part of this disclosure. It is to be understood that this invention is not limited to the specific devices, methods, applications, conditions or parameters described and/or shown herein, and that the terminology used herein is for the purpose of describing particular embodiments by way of example only and is not intended to be limiting of the claimed invention. Also, as used in the specification including the appended claims, the singular forms "a," "an," and "the" include the plural, and reference to a particular numerical value includes at least that particular value, unless the context clearly dictates otherwise. The term "plurality", as used herein, means more than one. When a range of values is expressed, another embodiment includes from the one particular value and/or to the other particular value. Similarly, when values are expressed as approximations, by use of the antecedent "about," it will be understood that the particular value forms another embodiment. All ranges are inclusive and combinable.

It is to be appreciated that certain features of the invention which are, for clarity, described herein in the context of separate embodiments, may also be provided in combination in a single embodiment. Conversely, various features of the invention that are, for brevity, described in the context of a single embodiment, may also be provided separately or in any subcombination. Further, reference to values stated in ranges include each and every value within that range.

In a first aspect, the claimed invention provides analysis devices. These devices suitably include a membrane having a thickness of from about 20 nm to about 100 nm having a thinned region with a thickness in the range of from about 1 to about 20 nm. The device suitably includes at least one pore extending through this region of the membrane. The pore suitably has a characteristic cross-sectional dimension (e.g., a diameter) in the range of from about 1 nm to about 1000 nm. The devices also suitably include a supporting layer adjacent to the membrane.

The membranes of the disclosed devices (shown as layer 3 in the attached figures) are formed of a number of materials. Nitrides, oxides, and the like are all considered suitable. Nitrides may be silicon nitride, boron nitride, titanium nitride, gallium nitride, and the like; silicon nitride is considered especially suitable. Suitable oxides include silicon oxide, hafnium oxide, titanium oxide, aluminum oxide, and the like. Membrane materials that can be processed by standard lithography techniques are considered especially suitable.

The membrane may also include a carbonaceous material, such as a carbide, graphite, graphene, diamond, and the like. Silicon, gold, silver, platinum, gallium arsenide, and the like are all suitable, as are polymeric materials. Polymeric materials useful in the claimed invention include polymethyl methacrylate, polystyrene, polyimide, and the like. The polymeric material may be used in the membrane, but may also be present as a supporting layer or a capping layer of the device. The supporting layer may be fabricated from any of these materials, and may suitably include silicon, germanium, gallium arsenide, glass, quartz, or alumina.

The thinned region of the membrane suitably has a thickness in the range of the sub-nanometer range, or from about 0.1 nm to about 20 nm, but may have a thickness in the range of from about 1 nm to about 15 nm, or even in the range of from about 2 nm to about 6 nm. The thinned region may have a cross-sectional dimension (e.g., diameter, width) in the range of from about 1 nm to about 1000 nm, or from 10 nm to about 100 nm, or from about 20 nm to about 50 nm. Larger thinned regions are within the scope of the present disclosure.

A supporting layer suitably has a thickness in the range of from about 1 micron to about 2000 microns, or even in the range of from about 10 microns to about 200 microns. The optimal thickness of the supporting layer will depend of the specific uses of the device, and the user of ordinary skill in the art will encounter little difficulty in fabricating a supporting layer of the proper thickness.

The pores of the devices suitably have a characteristic cross-sectional dimension (e.g., diameter) in the range of from about 0.1 nm (i.e., a sub-nanometer size) or even 0.5 nm to about 500 nm, or from about 1 nm to about 200 nm. The optimal pore size will be apparent to the user, based on their needs. As non-limiting embodiments, pores having a diameter of from about 1 nm to about 3 nm are considered suitable for analyzing ssDNA, and pores having a diameter of from about 2 nm to about 10 nm are considered suitable for analyzing dsDNA. DNA samples may have both ss and ds portions. The ratio of the membrane thickness to the thickness of the thinned region is in the range of from about 100:99 to about 100:1, or even 100:51 to 100:5.

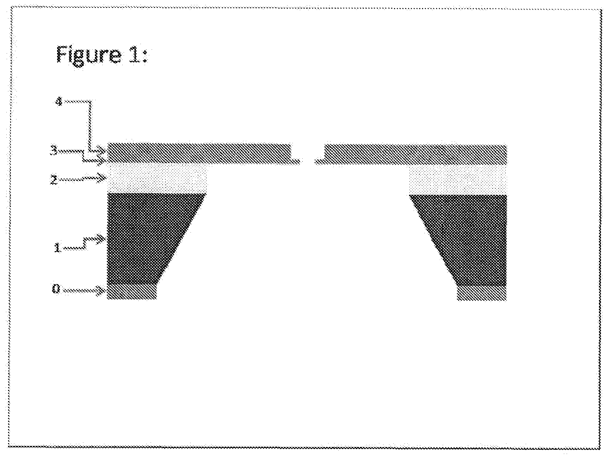

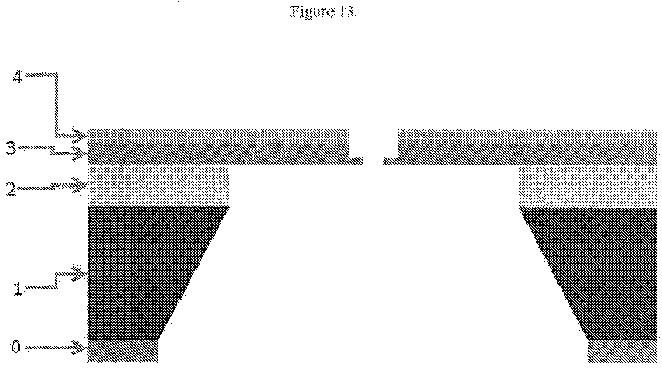

As shown in the attached figures, the nanopore may extent through the entire thickness of a membrane layer (Figure 1), or may extend through a locally thinned region of the membrane layer (Figure 13). The optimal configuration will depend on the user's needs.

As shown in figure 1, a device may include a base 0, a support 1, a dielectric 2, a thin membrane 3, and a upper membrane 4. A pore may be formed in the thin member 3, as shown in the figure. The thin membrane may have a thickness of only a few nanometers, or of tens of nanometers. The upper membrane 4 may have a thickness of a few nanometers, tens of nanometers, or even 100 - 1000 nanometers, depending on the user's needs. Because free standing (unsupported) membranes having a thickness of about 5 nm or less may experience mechanical instability or may not always be sufficiently robust for all applications. The devices shown in Figure 1 thus enable the user to overcome this challenge, as a pore is formed in the membrane 3, which membrane may have a thickness of 5 nm or less, but which membrane is reinforced by the upper membrane 4. A variety of materials are suitable for layers 0-4, which materials are described elsewhere herein in more detail.

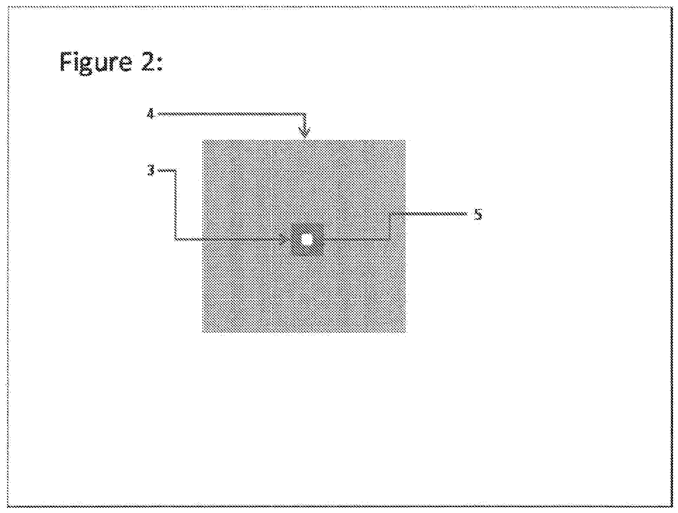

Figure 2 illustrates a top-down view of a device according to Figure 1. As shown in Figure 2, the upper membrane 4 includes a window that exposes thin membrane 3, in which membrane is formed a pore 3. As explained elsewhere herein, the pore need not be square; it can be of virtually any shape. The thin membrane need not necessarily include a pore. In such embodiments, the thin membrane may serve as a stage or support for microscopy or other application. As shown in Figure 40, the thinned membrane provides a reduced background for observing or measuring a sample; the sample of interest is deposited on the window substrate, and one or more of the optical methods above is used to image the sample. Some substrates have intrinsic fluorescence (e.g., SiN is known to be "noisy" for blue-green fluorescence measurements. The reduction of the thickness for local imaging reduces the background extinction or fluorescence from the substrate, enabling better signal and contrast from the sample of interest. A user may accordingly use such a device as a sample stage in a microscopy application.

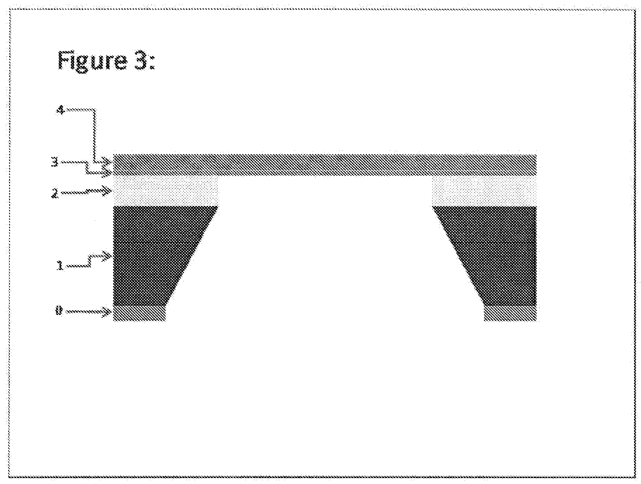

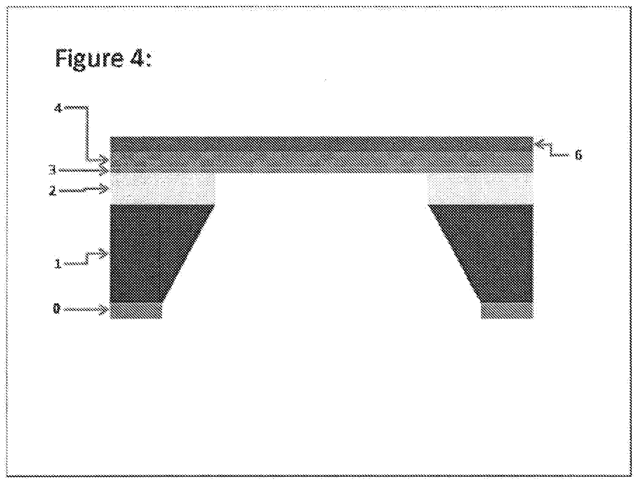

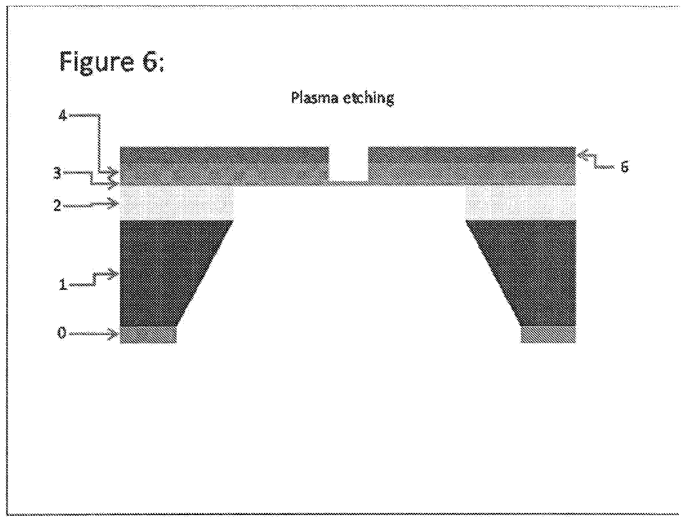

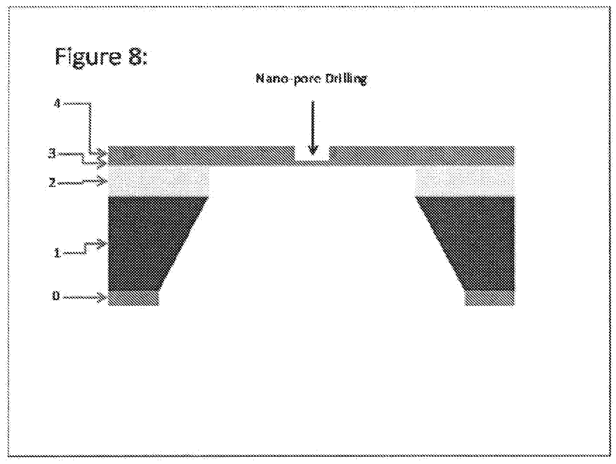

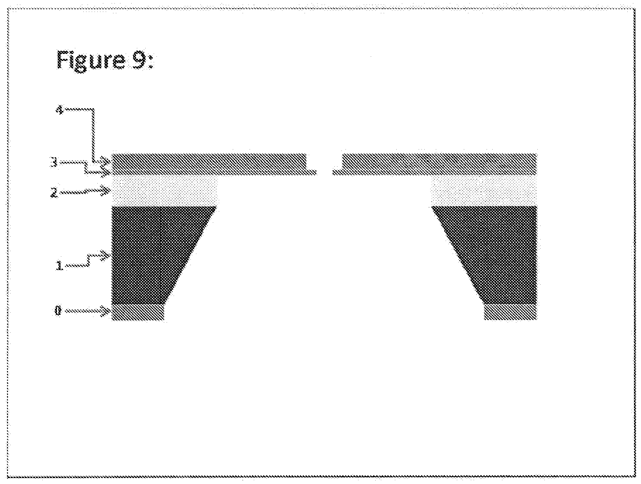

Figures 3-9 illustrate an exemplary fabrication process. As shown in Figures 3 and 4, a resist material 6 may be applied atop the upper membrane 4. The resist may be developed/removed to open a window in the resist that defines a region of the upper membrane 4. Plasma etching may be used to remove material from the upper membrane 4, which effectively defines a cavity or void above the thin membrane 3. The resist 6 may be removed (Figures 6-7). The user may then form a nanopore in the thin membrane 3 (Figure 8) to give rise to the completed device shown in Figure 9.

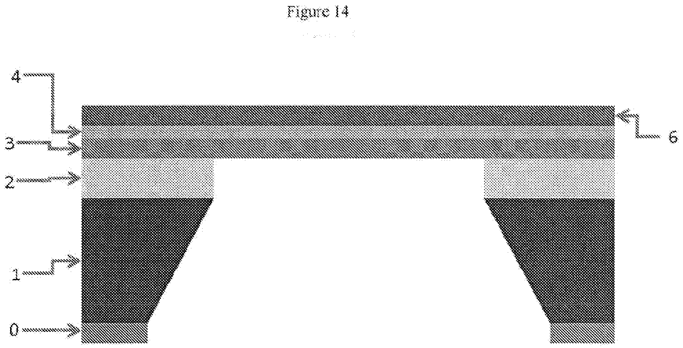

Figures 13-17 depict one non-limiting manner of fabricating the devices shown in Figure 13. Briefly, Figure 14 depicts a workpiece having resist (6), a capping layer (4), a membrane (3), a dielectric (2), a support (1), and an additional support (0) layer The support layer (0) is suitably a hard material, such as silicon. The support layer (1) is suitably silicon, GaAs, and the like. The dielectric (2) may suitably be silicon oxide or other dielectric material. The dielectric layer is optional, and is not necessary to the devices' function.

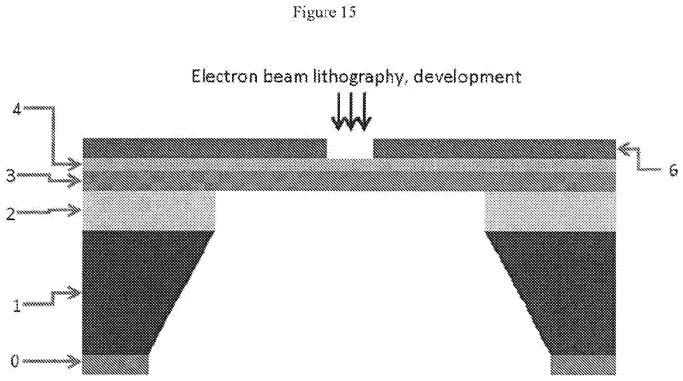

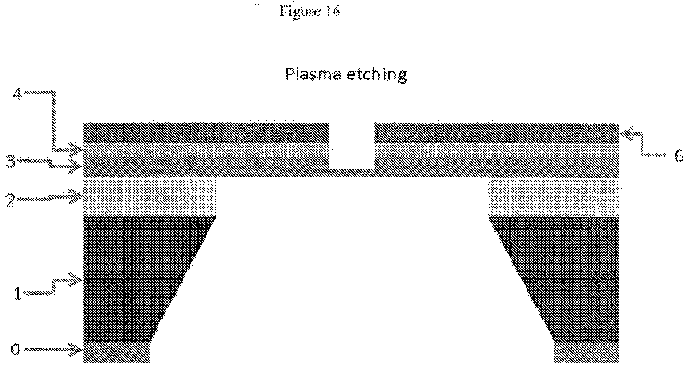

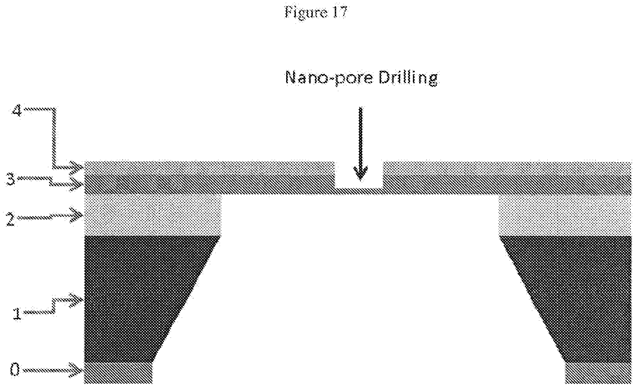

A portion of the resist is selectively removed (Figure 15), and then portions of the capping layer and the membrane layer are removed (Figure 16-17). The nanopore is then formed in the locally-thinned region of the membrane layer (Figures 17, 13).

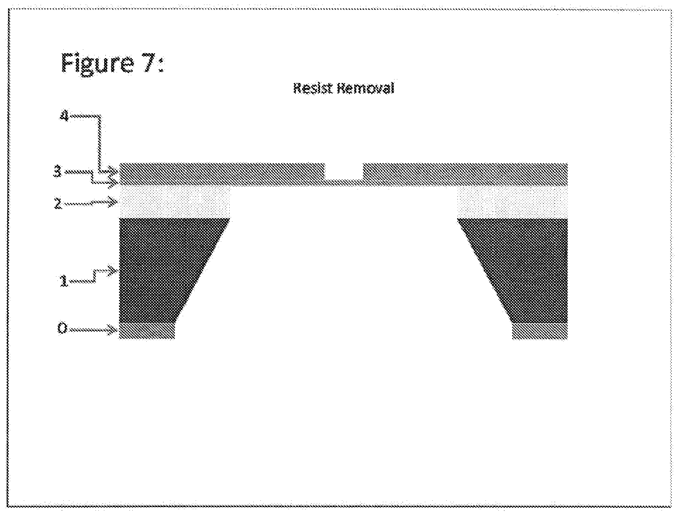

In some embodiments (Figures 1-9), the fabrication process entails first removing a portion of the resist (6) disposed atop the device workpiece via electron beam lithography (or other etching method) and corresponding development (Figures 4-5). Plasma etching, for example, is used to remove a portion of the capping layer (4) (Figure 6) to expose the comparatively thin membrane material (3). Resist (6) may be removed (Figure 7), and a nanopore may be formed - as described elsewhere herein - in the now-exposed membrane (Figures 8-9).

Figure 10(a) depicts another embodiment of nanopore device fabrication. As shown in that figure, PMMA resist (topmost layer) is removed via electron beam lithography. Reactive ion etching (RIE) and PMMA development then follows to thin local regions of the SiN membrane to a thickness defined by ($h$). A silicon oxide layer is disposed (in this figure) below the membrane, and a silicon support (bottom-most layer) is also present.

Figure 10(b) depicts AFM and optical microscopy images of a device according to the claimed invention. Following RIE process and lift-off, the AFM image of the 250 nm squares is shown in the inset (17 nm thinning). In this example, the thinning was checked for different RIE times and showed excellent linearity (slope: 1 nm/sec). In this way, the user can, by considering the etch rate, effect a desired degree of thinning in

The bottom right-hand images in Figure 10 show bright-field (BF) and annular dark field (ADF) images of the etched membranes, as well as corresponding images for a 10 nm pore (inset). The intensity profiles shown adjacent to the square-shaped pores highlight the exceptional contrast of ADF imaging, which is useful for accurate thickness determination.

A device may include single or multiple pores, as shown in Figure 10. Arrays or rows/strips of nanopores may be used. Arrayed pores are suitably spaced far apart enough from one another so as to avoid cross-talk between neighboring pores. Pores may be present in square (n x n) arrays. The arrays may include several pores, tens of pores, or even hundreds of pores, if the user so desires. The pores may be present in rows or strips, depending on the configuration of the device.

Pores are suitably circular or elliptical in conformation, but may also be polygonal. The pores may be square, pentagonal, rectangular, or other polygons having from 3 to 12 sides.

In some embodiments, a dielectric layer is disposed adjacent to the membrane. Without being bound to any particular theory, the dielectric layer improves the performance of the devices. The dielectric layer (layer 2 in the attached figures) suitably contacts the pore-bearing membrane where the supporting layer is present. Suitable materials for the dielectric layer include silicon oxide, aluminum oxide, silicon nitride, and the like. The dielectric layer, however, is optional, and need not be present.

In some embodiments, the devices include a capping layer (layer 4 in the attached figures). The capping layer is suitably disposed adjacent to the membrane. The capping layer material is suitably selected from the set of materials that are suitable for use in the membrane. In some embodiments, the capping layer and the membrane are formed of the same material.

The capping layer suitably includes an opening that overlaps, at least in part, a pore of the membrane. This opening may, in some embodiments, be used to assist the user in locating one or more pores of the membrane, as the pores are nanometer-scale. The opening suitably has a characteristic cross-sectional dimension greater than the corresponding cross-sectional dimension of the at least one pore of the membrane. The opening suitably has a characteristic cross-sectional dimension in the range of from about 10 nm to about 10 microns, or from about 50 nm to about 1 micron, or from about 100 nm to about 500 nm, or even about 250 nm. The opening in the capping layer may be circular, but can also be elliptical or polygonal in configuration.

The devices also may include a device capable of applying a gradient across the pore. Batteries, magnets, voltage generators, and the like are all considered suitable. The gradient may be an electrical gradient, a magnetic gradient, an ionic gradient, a pressure gradient, and the like. An electrolytic fluid is suitably present on both sides of the pore, so as to allow an electrical gradient to be passed across the pore to drive (or pull) a macromolecule across the pore. The fluid not necessarily be electrolytic, as non-conducting fluids may be useful where a pressure, magnetic, or other gradient is used to translocate the analyte across the pore.

The devices also suitably include a monitoring device capable of detecting a signal related to passage of a macromolecule across the pore, such as a change in electrical current related to passage of an analyte across the pore. The device may also be a device that detects an optical signal, such as a signal related to the passage of a fluorophore or other label that may pass through the nanopore.

The devices may further include a device (suitably a computer) capable of comparing the signal related to passage of a macromolecule across the pore to a signal evolved from passage of a macromolecule of known structure across the pore. In this way, the user may match the signal generated by a macromolecule of unknown structure to a signal generated by a macromolecule of known structure to determine the structure of the macromolecule being tested.

The devices may also include a device (e.g., a computer) that correlates a signal detected from the analyte or nanopore to a property of the analyte, the concentration of the analyte, or both. For example the device may correlate the number of passage events to the concentration of the analyte in the sample. This may be accomplished by comparing the number of passage events (or even the number of passage events per time) to calibration curve. The device may also output (e.g., display) the passage events, or may save a record of the events to a computer readable medium. The devices may display the signals detected at the pore as the signals are detected, i.e., in real-time.

Alternatively, the user may correlate the detected signal or signals to a property of the analyte. The user may do so by comparing a signal or signals evolved from the translocation of the analytes through the pores to a calibration curve or other standard.

Also provided are methods of fabricating devices. These methods include removing at least a portion of a first material disposed adjacent to a membrane material having a thickness in the range of from about 20 nm to about 200 nm so as to expose at least one target region of the membrane material; and etching at least a portion of the at least one target region of the membrane material so as to reduce the thickness of the membrane material within the target region to from about 0.1 nm to about 50 nm, or even from about 3 nm to about 30 nm. This can, in some embodiments, be conceptualized as thinning the targeted region of the membrane material.

The membrane may, in some embodiments, have a thickness in the range of even 5 nm to about 20 nm. The membrane may also have a thickness in excess of 200 nm, e.g., from about 200 nm to about 500 nm, or even to about 1000 nm. The first material - which may be a resist - may have virtually any thickness; the optimal thickness will depend on the user's needs and will be determined without undue experimentation.

The user may form at least one pore that extends through the target region of the membrane material. The pore may be formed by methods known in the art, e.g., application of an electron beam, a focused ion beam, heavy ion irradiation, chemical etching, or any combination thereof. The first material may be removed by electron beam lithography, application of etching or dissolution reagents, ion beams, and the like - suitable techniques will be known to those of ordinary skill in the art.

A variety of materials may be used as the first material. Polymethyl methacrylate, polymethyl glutarimide, styrene methyl acrylate, and the like are all suitable. Etching to reduce the thickness of the membrane may be accomplished by plasma etching, wet etching, focused ion beam etching, reactive ion etching, and the like. or any combination thereof.

The resist (first) material is suitably removed by electron beam lithography, optical lithography followed by development using an appropriate developing solvent, optical interference lithography followed by development using the appropriate developing solvent, nanoimprint lithography followed by development using the appropriate developing solvent, and the like. The resist suitably comprises polymethyl methacrylate, polymethyl glutarimide, styrene methyl acrylate, and the like; suitable resist materials will be known to those of ordinary skill in the art.

Resist material remaining after the first removal (Figure 7) may also be removed, if the user desires. A capping layer may be present adjacent to the membrane (pore-bearing) layer, and a portion of the capping layer may be removed (Figure 6) so as to expose the target region of the membrane material. This removal is suitably accomplished by wet-etching, dry etching, plasma etching, or any combination thereof.

Exemplary device fabrication is illustrated in Figure 34, Figure 35, and Figure 36. In Figure 34, a mask layer 0 (e.g., silicon nitride) is disposed adjacent to a silicon or other substrate material 1. A dielectric material 2 (e.g., silicon oxide) is disposed adjacent to a thinnable membrane material 3. The thinnable membrane material 3 may be silicon nitride. As shown in figure 35, a portion of the membrane 3 is removed so as to give rise to a thinned region of the membrane, which thinned region has a thickness smaller than that of the unthinned potion of the membrane 3. As shown in Figure 36, a pore is then formed in the thinned region of the membrane 3.

This technique thus enables formation of thin (or, short) pores in a thicker membrane material. In this way, the user may create advantageously thin pores - which have useful properties, as described elsewhere herein - in a comparatively thick membrane 3, which membrane lends structural rigidity to the device. Methods of forming pores are well-known to those of ordinary skill in the art. A reduced area of the thinned membrane of a sub-micron scale is useful (but not necessary). It may also be useful - but not necessary - to reduce the membrane thickness in patterned areas that are at a distance from the edge of the support of the larger membrane. This may reduce stress in the thin membrane that results from the interface between the freestanding membrane and the supported membrane.

Also provided are methods of analyzing an analyte. These methods include translocating at least a portion of an analyte (e.g., miRNA or a macromolecule) through a pore disposed in a membrane having a thinned region with a thickness in the range of from about 0.1 nm to about 20 nm and detecting a signal related to the translocation of the molecule through the pore.

Translocation is suitably effected by application of a gradient, such as an electrical field, a magnetic field, an ionic gradient, or any combination thereof. The thickness of the pore is suitably between about 0.1 to about 20 nm, or from about 1 nm to about 10 nm, or even from about 2 nm to about 5 nm. The optimal thickness of the pore will depend on the needs of the user and on the characteristics of the macromolecule being analyzed.

The user may monitor an electrical signal, a visual signal, or even some combination of these. Electrical signals - such as current - are considered especially suitable. Microscopy instruments are suitably used to gather optical or visual signals from the macromolecules under analysis. The detection of the signal may be accomplished by electrodes, as shown in the exemplary embodiments herein.

In some embodiments, correlating comprises comparing the signal related to the translocation of the macromolecule through the pore to a signal generated from the translocation of a macromolecule of known structure through a pore. For example, a user may know that a particular sequence of six bases yields a particular electrical current signal when that sequence is passed through a detector nanopore. If the user then observes that same current signal when analyzing a macromolecule of unknown structure, the user can conclude that the macromolecule undergoing testing has the six base sequence. The signal can also be used as a measure of the size of the molecule being analyzed, and the correlating aspect may thus include directly measuring the signal and correlating the signal to the size of the molecule being analyzed.

Also provided are additional analysis devices. These devices suitably include a first membrane having a thickness in the range of from about 5 nm to about 100 nm; and a second membrane disposed adjacent the first membrane, the second membrane having a thickness in the range of from about 2 nm to about 20 nm. The second membrane may include at least one pore extending therethrough, the pore having a cross-sectional dimension in the range of from about 1 nm to about 100 nm. The second membrane may include hafnium oxide, silicon oxide, titanium oxide, aluminum oxide, and the like. The first membrane suitably includes a cavity in register with the pore of the second membrane.

The construction of exemplary devices is shown in Figures 37-39. As shown in those figures, a thinnable membrane material 3 is disposed adjacent another membrane material 4. The membrane material 4 suitably has a thickness of a only one or a few nanometers, although thicknesses of 5, 10, 20, or even 30 nm are considered suitable. The membrane material 4 is suitably disposed adjacent to an insulator 2, which is in turn adjacent to a dye or support 1, which is in turn adjacent to a mask layer 0. The user suitably etches a cavity in the thinnable material 3 (as shown in Figure 38, so as to expose a region of the membrane material 4. The user may then form a nanopore (Figure 39) in the exposed region of the membrane material 4. In this way, the user may construct a thin (short) nanopore in a structurally robust assembly, which assembly has a rigidity conferred on it by the thinnable material 3. While the figures are not necessarily to scale, it is useful (but not necessary) to form the cavities and pores in regions that are relatively distant from the interface of the membrane 4 and the supporting material 2.

Further disclosed are detection devices. These devices suitably include a first capture material configured to bind specifically to a first molecule (e.g., miRNA); a membrane having a thickness in the range of from about 20 nm to about 100 nm having a thinned region thereon, the thinned region having a thickness in the range of from about 1 nm to about 20 nm, and a first pore extending through the thinned region, the first pore being in fluid communication with the capture material; and a detector configured to detect a signal related to passage of the first molecule through the first pore. Suitable membranes and pores formed therein are described elsewhere herein in further detail.

The capture material may include a protein, a porous support (e.g., a monolith), a bead, and the like. The porous support may be polymeric in nature. The capture material is suitably configured so as to bind to the first molecule (or to a molecule that is itself bound to the first molecule). For example, the capture material may include a protein - e.g., p19 - that preferentially binds to a probe-miRNA duplex, as shown in Figure 21(a). The capture material may include one or more nucleotides that bind to the first molecule or a species that is itself bound to the first molecule.

In some embodiments, the first molecule is bound - as shown in Figure 21a - to a probe that is specifically complementary to that molecule. Such a probe may be a nucleotide-containing probe that includes a nucleic acid sequence that is complementary to a sequence on the first (target) molecule. The capture material may be selected to as to bind preferentially to a predetermined first miRNA molecule.

In some embodiments, the devices include a second capture material. This second capture material may be one that binds preferentially to a second miRNA molecule that differs in at least one aspect (e.g., size, nucleic acid sequence) from the first miRNA molecule. In some embodiments, the user may contact a sample with a second probe that is complementary to a second molecule (e.g., a miRNA molecule) that differs in some respect from a first miRNA molecule.

The capture material may be positioned such that it is in fluid communication with a pore. The first and second capture materials may be in fluid communication with the same or different pores. This may be accomplished by using different channels to connect the different capture materials to different probes. The devices may, of course, include 3 or more different capture materials, channels, or even pores. By utilizing multiple channels or pores, the devices may perform multiplexed analysis or detection for multiple analytes.

The devices may also include a sample storage chamber or input chamber. This chamber may be placed into fluid communication with the first capture material. A valve, septum, or other fluidic element may be used to modulate flow between the input chamber and the capture material.

The present disclosure also provides additional detection devices. These devices suitably include a first capture material that binds specifically to a first molecule, a first membrane having a thickness in the range of from about 5 nm to about 100 nm; and a second membrane disposed adjacent the first membrane, the second membrane having a thickness in the range of from about 2 nm to about 20 nm, and the second membrane having at least one pore extending therethrough, the pore having a cross-sectional dimension in the range of from about 1 nm to about 100 nm, and the first membrane having a cavity formed thereon, the cavity being in register with at least one pore of the second membrane, the first pore being in fluid communication with the first capture material; a device configured to apply a gradient across the pore; and a detector configured to detect a signal related to passage of a molecule through the first pore.

Suitable membranes and pores are described in additional detail herein, as are suitable capture materials. An exemplary membrane/pore device is shown in Figures 37-39, which figures show a nanopore formed in a thin layer 4, the thin layer 4 being supported by a mask layer 0, a dye 1, and dielectric insulator 3. As shown in those figures, a cavity is formed in the membrane material 3 in register with the pore formed in layer 4.

These devices are considered especially suitable for miRNA detection. The devices are also useful for detection of DNA, RNA, and other biological entities. The sample may be present in a storage chamber (e.g., the input chamber shown in Figure 33), which chamber may be in fluid communication with a capture material. The devices may include more than one capture material, and the configuration of the capture materials and pores is described herein in connection with the other disclosed devices.

The present disclosure also provides methods of detecting an analyte. These methods include contacting a sample to a first capture material that preferentially binds to a first analyte, eluting the first analyte from the capture material, translocating the first analyte through a first pore disposed in a thinned region of a membrane, the thinned region having a thickness in the range of from about 0.1 nm to about 20 nm; and detecting a signal related to the translocation of the analyte through the first pore.

The first analyte may be miRNA, DNA, RNA, tRNA, and the like. Biological analytes are considered especially suitable for the provided methods. The signal evolved from the translocation of the analyte (e.g., a molecule, such as miRNA) through the pore. Electrical signals, such as current, are suitably monitored.

In some embodiments, the capture material preferentially binds the analyte. In other embodiments, the capture material preferentially binds an analyte-probe combination or duplex. As shown in Figure 21a, the user may contact a target molecule with a probe that binds specifically to the target. The probe-target duplex then binds to a capture material, such as p19 or other protein. The capture material may be part of a bead or a porous support. The beads may be of virtually any size; they may be of sub-micron size, micron size, tens of microns, or even hundreds of microns or even millimeter-scale in size. Washing may be performed to remove other analyte (e.g., unbound miRNA) from the mixture. The hybridized probe:miRNA comple may then be eluted from the capture material, by application of salt, heat, or other reagents that effect elution.

In some embodiments, the user may contact the analyte-containing sample to a second probe. This second probe is suitably selected so as to bind preferentially to an analyte (e.g., second analyte) that differs from the first analyte. The user may then bind the second analyte to a capture material, wash away other analytes, and elute the second analyte for detection by the pore, where the user detects a signal related to the translocation of the second analyte through the second pore.

The foregoing may also be accomplished by translocating an analyte through a first pore formed in a first membrane. The first membrane is suitably disposed adjacent to a second membrane; the second membrane having a thickness in the range of from about 5 nm to about 100 nm and the second membrane having a cavity formed thereon, the cavity being in register with the first pore, the first membrane having a thickness in the range of from about 2 nm to about 20 nm, and the first pore extending through the first membrane, the pore having a cross-sectional dimension in the range of from about 1 nm to about 100 nm, the first pore being in fluid communication with the first capture material; and detecting a signal related to the translocation of the molecule through the pore.

Exemplary Embodiment

The following is an exemplary, non-limiting embodiment of the claimed invention. The specific materials and techniques recited here are exemplary only and should not be taken as limiting the scope of the invention to these particular materials and techniques.

The present invention presents a unique approach for fabricating sub-10 nm thick membrane devices, as well as the use of such membranes for nanopore-based nucleic acid analysis. This innovation involves, inter alia, the steps of (1) electron-beam lithography is used to expose a sub-micron region on a 50 nm-thick SiN window, (2) reactive ion etch is used to locally thin the exposed SiN region with sub-nanometer control, (3) the electron-beam of a transmission electron microscope is used to drill a nanopore in the thinned SiN region. Thereafter, the nanopore devices are treated using established protocols for subsequent biomolecular analysis. Analysis of macromolecular translocation through solid-state nanopores is described in, *e.g.,* Wanunu et al., Biophys. J., August 2008, vol. 95, and Wanunu et al., Nature Nanotechnology, Vol. 5, November 2010.

An exemplary, non-limiting fabrication scheme is shown in Figure 1 (plan view) and Figure 2 (top view). The ultrathin nanopore is supported by, e.g., a ca. 500 μm thick <100> p-type silicon (Si) wafer (layer 1 in Figure 1), which may contain a several μm thick thermal oxide on one or both of its sides (layer 2 in Figure 1).

Using low-pressure chemical vapor deposition (LPCVD), ca. 40 nm of low-stress SiN is deposited on both sides of the wafer. Standard photolithography followed by anisotropic etch using KOH is used to divide the wafer into a square array of 5 x 5 mm$^2$ chips, as well as to define in each chip a free-standing square SiN window with dimensions ranging from 1x1 to 500 x 500 μm$^2$. If oxide is present in the free-standing window, the oxide is removed by treatment with hydrofluoric acid or by buffered oxide etch using standard protocols. This yields a workpiece as shown in Figure 3.

The top side of the substrate in Figure 3 is then spun-coated with an electron beam resist C2 950 PMMA (950 molecular weight polymethyl methacrylate, 2% in chlorobenzene) at 5000 rpm for 50 seconds to achieve a resist layer of ~ 100 nm thickness, and then baked on a hotplate at 180°C for 10 minutes (Figure 4).

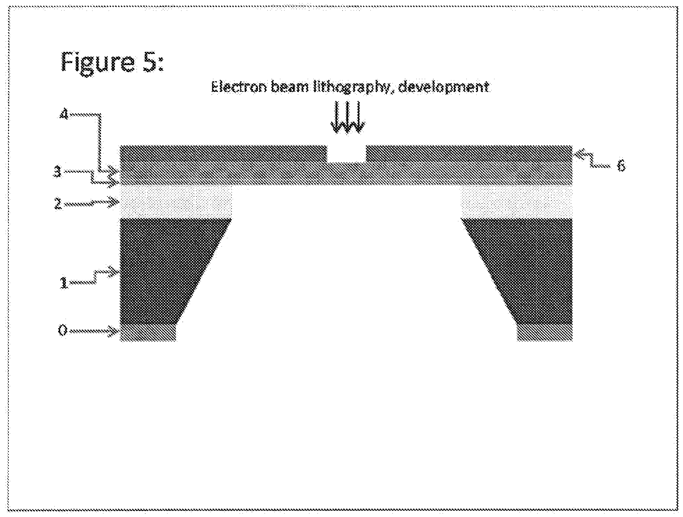

Electron beam lithography is then used to write a square of 50x50 to 1000x1000 nm2 onto the resist-coated SiN window, using a 20 pA electron beam of 50 kV (Elionix 7500-ELS) and a beam dose of 750 μC/cm$^2$. The device is then developed in 1:3 volume ratio of MIBK (methyl isobutyl ketone) and isopropanol, respectively, for 60 seconds (Figure 5).

The exposed areas of the SiN are further thinned by SF$_6$ plasma etching using a 50 watt RF source and 0.4 mtorr SF6 chamber pressures (Figure 6). The duration of the RF source determines the etch depth, which is suitably 1 nm per second under the indicated conditions. Other etch rates are within the scope of the claimed invention, and the optimal etch rate will depend on the user's needs and capabilities. The etch rate can be modulated or chosen so as to enable the user to achieve devices of the desired configuration.

In another, non-limiting embodiment, the user etches a SiN layer (shown as layer 4 in the attached figures) so as to expose a layer (layer 3) of hafnium oxide. The hafnium oxide may have a thickness of from about 2 nm to about 5 nm, in some embodiments.

Atomic force microscopy is used to accurately measure the etched depth in a given batch of etched chips. This process can be simultaneously carried out on a set of 1-300 chips. Following the thinning, PMMA resist is removed by incubation in warm acetone, yielding a device shown in Figure 7.

Each chip is then inserted into a transmission electron microscope equipped with a field emission gun (e.g., a JEOL model 2010F) and a nanopore in the range 1-100 nm is drilled by focusing the electron beam onto the thinned region of the membrane using previously reported protocols (see Figure 8), yielding a nanopore device as shown in Figure 9. Formation of nanopores in membranes is well-characterized in the art, and is described in, *e.g.,* Storm et al., Nature Materials, August 2003, vol. 2, the entirety of which is incorporated herein by reference for all purposes.

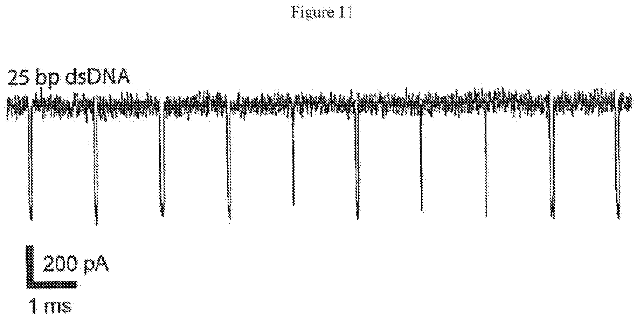

Figure 11 illustrates the detection of 25 bp dsDNA using a 3 nm solid-state nanopore according to the claimed invention in a 20 nm thick SiN membrane (T = 0° C., V = 300 mV). In the figure, concatenated single-molecule traces are shown; the small pore and low temperature combination facilitates detection of these short molecules (mean transport time = 80 microseconds), which is an important step for detecting RNA-drug complexes in design-RNA sequences, such as the ribosomal A-site and the TAR site of HIV RNA; and

The improved resolution achieved by the claimed invention is shown by Figure 12, which figure illustrates DNA translocation through 4 nm pores in SiN membranes of various thickness values.

Figure 12a shows concatenated events for 3 kbp dsDNA translocations, as a function of membrane thickness ($h$) (V = 300 mV, T = 21°C., 1M KCl, pH 8). As shown at the top of the figure, the detected current increases with decreasing $h$ values, such that a nanopore having a thickness of 8 nm is more than four times more sensitive than a nanopore having a thickness of 60 nm. These data underscore the performance advantages achieved by the thinned membranes and thin (low-$h$) nanopores of the present disclosure.

Figure 12b shows expanded events from the traces shown in the upper quadrant of the figure. As shown, nanopores having a thickness of 8 nm enable the user to resolve individual translocation events at a far greater resolution than nanopores having thickness values of 15, 25, or even 60 nm. Figure 12c shows semi-log all-point current histograms from the events shown at the top of the figure, illustrating the ability of the thinned nanopores to resolve DNA translocation at improved resolution.

Figure 12d illustrates the relationship average experimental $<I_o>$ (circles) and the most probable DNA current amplitude $\Delta I_p$ (triangles) on $h$. The dashed line among the circles is a fit using Eqn. 1 to the average $I_o$ data from combined data of ~20 pores, which yields an effective pore thickness $h_{eff} = h/(3.04 \pm 0.30)$ ($h_{eff}$ scale shown on top x-axis). The fit to $\Delta I_p$ values (dashed line with triangles) is based on a geometric model described in detail herein. The inset shows $\Delta I_p < I_o>$, which did not change appreciably with $h$. The dashed line in the inset is the ratio of the fits to $\Delta I_p$ and $<I_o>$ from the main plot.

Figure 12e illustrates the signal-to-noise ($S/N$) and mean transport time as a function of $h$ ($h_{eff}$ shown on top x-axis). $S/N = \Delta I/I_{RMS}$, where $I_{RMS}$ at 100 kHz bandwidth is $75 \pm 5$ pA. Mean transport times were obtained from the dwell-time distributions.

Thinned pores presents many advantages, including improved signal-to-noise (improving from when the nanopore thickness goes from 60 nm to 8 nm), as well as improved resolution by contraction of the resistive sensing zone to a few nm, which contributes greatly to RNA-drug complex detection and footprinting.

Without being bound to any single theory of operation, the advantage of the disclosed thinned nanopores can be described by reference to existing, thicker nanopores. As a non-limiting example, if a DNA molecule enters a pore with a thickness of about 20 nm, approximately 40 to 50 base pairs would reside within the pore at a given moment. If, however, the pore is reduced in thickness such that only 5-6 base pairs reside within the pore at a given moment, the detected current (or other signal) related to the DNA passage across the pore would be averaged over far fewer bases than where the pore accommodates 40-50 base pairs.

Ultrathin nanopores have been fabricated and were evaluated for their performance in DNA analysis. The locally thinned SiN membranes offer tremendous advantages, such as greater signal-to noise values and improved resolution, with similar DNA transport velocities as with thicker membranes. This method can be applied on a wafer scale, enabling the production of hundreds of substrates at a time, essential for success in this proposal.

Microscopy

In another aspect, the present invention provides stages or platforms suitable for high-resolution microscopy. These stages or platforms suitably include a membrane having a locally thinned region (e.g., Figure 13 and Figure 6), which thinned region confers enhanced microscopy properties on the membrane. The thin membranes may be used as sample stages or supports. These are especially suitable for supporting cells, cell contents, or other biological entities for observation.

As one example, the thin membranes may be used in Transmission Electron Microscopy (TEM) -based and scanning TEM (STEM) -based imaging and analysis, which includes: bright field imaging, annular dark field (ADF) and high-angle annular dark field (HAADF) imaging, electron diffraction analysis, electron energy loss spectroscopy (EELS), and energy dispersive spectroscopy (EDS). One advantage presented by the thin membranes are that the ultrathin support enables higher contrast (relative signal) from samples deposited onto the ultrathin region, then the contrast from sample deposited onto a thicker region. An additional advantage is that the wide choice of materials suitable for the ultrathin membranes enables elemental spectroscopy (e.g., EELS and EDS above) with little or no interference from the substrate.

The thinned membranes are also suitable for Scanning Electron Microscopy (SEM) -based imaging, which includes imaging using a secondary electron detector, backscattering detector, a transmission detector, and a so called STEM detector. The ultrathin substrates exhibit an extremely low backscattering cross-section, which results in the substrates being practically SEM-invisible. Such devices exhibit a much better contrast than normal (thick) counterparts, enabling better contrast and EDS signal. This is illustrated in figure 40, which figure illustrates (from left to right) a series of zoomed images of a 6 nm thinned membrane formed in a thicker support. (The thinned membrane also includes a pore formed therethrough; the pore is shown by the bright spot at the edge of the thinned region.

The membranes are also advantageous for optical microscopy, which includes wide field white light imaging, or confocal fluorescence, or epifluorescence, or total-internal reflection mode fluorescence, or luminescence mode (in which light from a light-emitting source is collected). In such applications, the sample of interest is deposited on the window substrate, and one or more of the optical methods above is used to image the sample. Many substrates have intrinsic fluorescence (e.g., SiN is known to be "noisy" for blue-green fluorescence measurements. The reduction of the thickness for local imaging reduces the background extinction or fluorescence from the substrate, enabling better signal and contrast from the sample of interest.

Accordingly, the microscopy supports suitably include a membrane material having a first region of from about 0.5 nm to about 20 nm in thickness and a support layer disposed adjacent to the membrane material. The membrane and support layers may include the materials described elsewhere herein, e.g., silicon nitride and the like. The first region may have a thickness of from about 2 nm to about 12 nm, or even from about 6 nm to about 10 nm.

Also provided are methods of fabricating a microscopy supports. These methods suitably include removing at least a portion of a material disposed adjacent to a membrane so as to expose a first region of the membrane. This is shown by, e.g., Figures 4-7, which figures illustrate removal of material adjacent to a membrane (3) having the claimed thickness. Figure 7 illustrates a device wherein a thin, lower membrane is exposed by formation of a cavity or other void in the material atop the lower membrane. The thin, exposed lower membrane may be used as a stage or support for samples undergoing optical microscopy, TEM, STEM, and other similar measurements. A pore may be formed in the lower, thin membrane, as shown in Figure 8.

In some embodiments, the methods include etching at least a portion of the first region of the membrane material so as to reduce the thickness of the membrane material within the target region. This is shown by, e.g., Figures 10 and 14-16, which depict removal of a material adjacent to the membrane and then etching of the membrane material (3) itself so as to thin a localized region of the membrane.

ADDITIONAL DESCRIPTION

In one exemplary platform described herein, a target microRNA is first hybridized to a probe; this probe:microRNA duplex is then enriched through binding to the viral protein p19; and, lastly, the abundance of the duplex is quantified using a nanopore. Reducing the thickness of the membrane containing the nanopore to 6 nm leads to increasing signal amplitudes from biomolecules, while reducing the diameter of the nanopore to 3 nm allows the detection of and discrimination among small nucleic acids based on differences in their physical dimensions. This approach detects picogram levels of a liver-specific miRNA from rat liver RNA.

**Forming Nanopores in Substrates**

Figure 18a depicts an exemplary thin solid-state molecular counter. In this embodiment, a region of a free-standing SiN membrane supported by a Si chip is thinned, after which a nanopore is fabricated using a transmission electron microscope (TEM). Biomolecular translocations (depicted in the inset) through the nanopore appear as transient reductions in the ion current. A TEM image of a ca. 4 nm diameter pore in a ca. 6 nm thick membrane is shown in the inset. As shown in the figure, an electrical gradient is applied across the pore so as to drive the analyte through the pore for detection.

The reduction of membrane thickness $h$ is described in Figure 18b. An optical micrograph of a processed membrane is shown in Figure 1c, in which a pattern of 1, 4, and 9 squares of different sizes has been exposed and etched. An AFM image of the thinned 3x3 array is shown in the inset. Knowledge of the initial membrane thickness and the etch depth allows calculation of the resulting membrane thickness. AFM characterization of the depth vs. etch time reveals an etch rate of 1.0 nm/sec, which is illustrated in Figure 18c inset and Figure 22.

The intrinsic fluorescence of SiN membranes from embedded nano-Si structures was also measured. Figure 18d shows that fluorescence background is reduced in the thinned regions, consistent with a reduced number of fluorescent structures. The thinnest nanopores were fabricated in 6 nm thick membranes (see Figure 12d), which are comparable in thickness to lipid bilayers. Nanopores may, as described herein, be fabricated in membranes that are thinner and thicker than 6 nm.

To characterize the sub-10 nm thick nanopores, bright-field (BF) and annular dark-field (ADF) scanning transmission electron microscopy (STEM) images of a 4.5 nm diameter pore in a 7 nm thick membrane are shown in Figure 19. Both BF and ADF STEM provide contrast that is very sensitive to membrane thickness. In BF-STEM, the thinned membrane region is a brighter area. The uniformity of the etched region is indicated by the line profile intensity (line in Figure 19a). A zoomed-in ADF-STEM image reveals a sharp drop in the intensity near the pore (line in Figure 19b). The contrast is reversed from BF to ADF, so that the pore appears dark.

Based on previous TEM and ion conductance measurements and the ADF-STEM measurements, nanopore shapes deviate from a perfect cylinder. However, a simplified geometric model using an equivalent cylinder of reduced thickness and equal diameter to the measured pore diameter can quantitatively explain the data. This reduced thickness is defined as the effective pore thickness $h_{eff}$, as illustrated in Figure 12a.

To a first approximation, $h_{eff}$ can be used to quantitatively explain electrolyte transport through the pore. Systematically changing the membrane thickness $h$ by controlled thinning should have a predictable influence on electrolyte transport through a pore fabricated in such a membrane. When voltage $V$ is applied, the ion current $I_0$ through a cylindrical pore with diameter $d$ and thickness $h_{eff}$ is approximated in high ionic strength solutions (>100 mM) by:

$$I_0 = V \left( \left[ \mu_{K^+} + \mu_{Cl^-} \right] n_{KCl} e \right) \left( \frac{4 h_{eff}}{\pi d^2} + \frac{1}{d} \right)^{-1} \qquad Eqn.\ 1$$

where $\mu$ is the electrophoretic mobility of a species, $n_{KCl}$ is the number density of KCl, and e is the elementary charge. This equation includes the access resistance, which dominates the conductance in the limit of $h_{eff} \rightarrow 0$. Passage of analytes through the pore transiently reduces the ion current because the ion flux is hindered. Therefore, for similar diameter pores, reducing the pore thickness should yield two experimental outcomes, increased $I_o$ (see Eqn. 1), and increased difference $\Delta I$ between the open pore current and the current upon occlusion with biomolecules.

To test this, one may use similar TEM conditions to fabricate dozens of 4 nm diameter pores in membranes with $h$ = 6 - 60 nm. Figure 12a shows a set of ~200 concatenated translocations of 3 kb linear double-stranded DNA (dsDNA) molecules for 4 nm diameter pores of different thicknesses, recorded at 21°C and 300 mV. Upon decreasing $h$, it is founds that: (1) open pore currents increase, and (2) amplitudes of the DNA translocation signals increase. Decreasing $h$ vastly improves the signal from biomolecules, as illustrated by the close-up view of representative events in Figure 12b. All-point current histograms from the traces shown in Figure 3a are plotted on a semi-log scale in Figure 12c. The histograms were normalized by subtracting the mean open pore current $<I_o>$ from each distribution. As predicted, the most probable current amplitude $\Delta I_p$ (dashed white lines) increases as $h$ decreases. Also, the broadness of the $\Delta I$ distributions is augmented in thinner pores. Broad $\Delta I$ distributions were previously observed for pores in lipid bilayers and solid-state membranes, and are likely a result of the varying transport speeds, interactions, and/or initial configurations of each molecule prior to translocation.

In Figure 12d is shown experimental mean open pore currents $<I_o>$ (circles) and the most probable DNA current amplitudes $\Delta I_p$ (triangles). Both quantities increase with decreasing $h$. The dashed line among the circles is a fit of $<I_o>$ based on Eqn. 1, where $h_{eff}$ is a fitting parameter. Using $d$ = 4.0 nm, $\mu_{K+}$ = 6.95x10$^{-8}$ m$^2$V$^{-1}$s$^{-1}$, and $\mu_{Cl-}$ = 7.23x10$^{-8}$ m$^2$V$^{-1}$s$^{-1}$, a best fit to the data is obtained using $h_{eff}= h/(3.04 \pm 0.30)$, in good agreement with previous measurements for thicker membranes (see top x-axis of Figure 12d). Based on the fit, the thinnest pores shows here with $h$ = 6 nm have a calculated $h_{eff} \approx 2$ nm.

To fit the experimental $\Delta I_p$ values, one uses a geometric model to compute the residual current through the pore when occupied by a DNA strand (see Figure 23 and related discussion). In this model, dsDNA is assumed to be a cylinder 2.2 nm in diameter, and $\Delta I$ is computed from the unobstructed area of the DNA-occluded pore. Once can add to this model a parameter S that describes the fraction of chloride ions excluded from the nanopore vicinity by the highly charged DNA coil. A previous report that $\Delta I$ increases as a function of DNA length in 4 nm pores suggests that the highly charged DNA coil excludes Cl$^-$ from the pore vicinity, therefore reducing [Cl$^-$] near the pore. Using a single parameter of 20±5% Cl$^-$ exclusion for 3 kb DNA, the results match the model for $\Delta I$ (Eqn. 2 herein) for all tested $h_{eff}$ values (see dashed curve). The inset to Figure 12d shows the experimental (markers) and calculated (dashed line) blockage fractions $\Delta I_p/<I_o>$ as a function of $h$. In the regime where access resistance does not dominate, i.e., for $h$ >10 nm, $\Delta I_p/<I_o>$ is independent of $h$.

Figure 12e displays signal-to-noise ratios $S/N \square \Delta I_p/I_{RMS}$ as a function of $h$, where $I_{RMS}$ is the open pore current RMS at 100 kHz bandwidth. $I_{RMS}$ is independent of $h$ ($I_{RMS}$ ~70-80 pA at 100 kHz bandwidth), which results in increasing $S/N$ values with decreasing $h$ because $\Delta I$ increases with decreasing $h$. $S/N$=46 for h=6 nm, a marked improvement over $S/N$~10 in similar diameter pores with h=25 nm.

Finally, a potential concern in this study was that reducing $h$ would also reduce DNA interactions with the pore surface, thereby speeding up DNA transport and presenting detection challenges. The right axis of Figure 12e shows that the mean transport times for the 3 kb DNA molecules are approximately independent of $h$, with less than 30% decrease in transport times when $h$ is reduced from 60 nm to 6 nm. The weak dependence of transport times on $h$ indicates that parameters other than surface interactions, for example, electro-osmotic drag, influence the transport dynamics.

**Discrimination among small nucleic acids**

Using the increased signal amplitude in thin nanopores, thin pore (d=3 nm, h=7 nm) were tested to discriminate among small nucleic acids. First were compared 22-bp RNA and 25-bp DNA, because the two molecules have similar effective solvation volumes (~35 nm$^3$ and ~32.3 nm$^3$, respectively) and molecular weights (~15 kD), while differing in cross-sectional areas by ~35% due to their different helicities (see in Figure 20a PDB files 1RPU and 2BNA from http://www.rcsb.org). Continuous current traces, as well as magnified sets of representative events are shown in Figure 20a. To quantify the difference between the molecular signatures, also shown are all-point current histograms for DNA and RNA , which highlight the characteristic signal from longer events. The histograms reveal peaks at characteristic current amplitudes of $\Box I_{DNA}$=0.54 nA and $\Box I_{RNA}$=0.92 nA. Similar amplitudes were obtained for 10-bp DNA and 25-bp DNA, although the 10-bp molecule often stuck to the pore for >1 ms timescales because the 10-bp molecule is comparable in length to the pore dimensions.

The difference in current amplitudes between short DNA and RNA is ~40%, in good agreement with their cross-sectional area differences. The mean transport times for the RNA molecule (50 $\mu$s) was significantly longer than the DNA molecule (20 $\mu$s). This, in addition to a broader $\Delta I$ distribution for RNA may be due to drag forces on the wider helical structure of RNA, and a greater extent of interactions between RNA and the nanopore. The differences in current amplitudes between DNA and RNA persist even for events with ≤16 $\mu$s duration, for which the signal amplitude is attenuated by as much as 20% due to use of a rank 1 median filter. Using a discrimination threshold of $\Delta I_{Thres}$=675 pA, one can discern DNA from RNA with >97% certainty by considering events with ≥16 $\mu$s durations, which represent >75% of the total detected events. Analysis of events with duration of ≤16 $\mu$s shows that the signal is attenuated by the same factor for both DNA and RNA, enabling discrimination even for events with this duration range. It was also checked whether discrimination based on $\Box I$ is not a result of the faster mean transport times for DNA by comparing events for DNA and RNA with similar durations, in which <10% overlap in the two $\Delta I$ distributions is seen.

The pores could also distinguish linear nucleic acids from more complex structures. For example, transfer-RNA (tRNA) is structurally more complex than duplex RNA and DNA due to the presence of unpaired bases and loops, which give rise to a bent structure (see in Figure 20a the structure of phenylalanine tRNA from PDB file 4TNA). The traces in Figure 20a shows events with amplitudes of 1.8 nA for the tRNA, a factor of 2 greater than for the 22-bp RNA molecule (0.92 nA). The mean transport time of the tRNA molecule is 1.04 ms, much longer than that of the linear nucleic acids (see expanded events in Figure 20a). These differences in signals between the three molecules show that one can discriminate among populations of different nucleic acids with certainty.

The disclosed devices and methods are, as described elsewhere herein, considered especially suitable for detection and/or quantification of miRNA. The devices may be used to detect miRNA that has a length of from 1 to about 10 base pairs, or from 1 to about 20 base pairs, or from 1 to about 50 base pairs, or even from 1 to about 100 base pairs. miRNA molecules may be 22 base pairs in length, which size is well within the capabilities of the disclosed devices and methods to detect or otherwise analyze.

Figure 20b shows mean capture rates vs. voltage for 25-bp DNA and 22-bp RNA (see Figure 29 and associated text). The mean capture rates are exponentially dependent on voltage in the range of 300-600 mV (curves are exponential fits to the data), suggesting that nucleic acid capture is a voltage-activated process. Based on the ratio of the observed event rate and the calculated arrival rate of molecules to the pore at 600 mV, one can estimate that >50% of the molecules that arrive at the pore are captured and detected.

The concentration of a sample can be measured from the frequency of molecular signals, provided that a calibration curve of capture rate vs. concentration is constructed. In Figure 20c, a log-log plot of the mean capture rate vs. concentration of 25-bp DNA is shown (see Figure 30 and associated text). The power-law fit to the data (line between data points) yields an exponent of 1.05 $\pm$ 0.03, indicating linearity over three orders of magnitude in concentration. This dynamic range can be extended by orders of magnitude by increasing the measurement time and applied voltage, or by adjusting the electrostatic potential at the pore entrance.

**Electronic platform for detection of specific microRNAs**

Electronic detection of small RNAs using a solid-state device may provide an alternative to existing methods for rapid and sensitive miRNA analysis. Microarray-based miRNA profiling, fluorescence, radioactive gel electrophoresis, and other novel techniques can detect sub-femtomole RNA levels, but none of these offer the unique aspects of nanopore detection, such as electronic sensing, single-molecule sensitivity, reusability, use of an unlabeled probe, and avoidance of surface immobilization. To detect a specific miRNA with a nanopore, a sequence-specific enrichment step of a particular miRNA may be useful (but not required), as miRNAs are <1% in concentration relative to other cellular RNAs.

To enrich a specific RNA, the p19 protein from the Carnation Italian ringspot virus was used. P19 binds 21-23 bp dsRNA in a size dependent, *sequence independent* manner. The p19 protein does not bind ssRNA, tRNA or rRNA.

To enhance isolation of the bound dsRNA C-terminal fusion of p19 with the chitin binding domain (CBD) was created, allowing linkage of the p19 fusion protein to chitin magnetic beads. The magnetic beads simplify the washing steps required to remove unbound RNA. Using p19 beads have achieved over 100,000-fold enrichment of the probe:miRNA duplex from total RNA. The protocol shown in Figure 21a for nanopore-based miRNA detection was used. This begins with isolation of total RNA from tissue and hybridization to a miRNA-specific oligonucleotide RNA probe fully complementary to a target miRNA. In step (I), the probe-hybridized total RNA is incubated with the p19 protein immobilized on magnetic beads, followed by washing to remove the remaining RNA. In step (II), the purified probe:miRNA duplex is eluted from the p19 protein. In step (III), the duplex is detected using a nanopore (see Figure 31 and associated text). A miRNA enrichment protocol may take several hours starting from isolated cellular RNA.

MiRNAs isolated with p19 may contain agents that interfere with nanopore detection of the probe:miRNA duplex. Along with the eluted duplex, other agents may be present, such as bovine serum albumin (BSA) that coats the beads, SDS used for dsRNA elution from the p19, and trace amounts of other RNAs. To eliminate the possibility of an artifact signal caused by these interfering agents, several controls were performed. The results are summarized in Figure 21b showing 30 second current traces for different samples, all of which have been treated using p19 beads.

The first trace shown at the top of Figure 21b is for the probe:miR122a duplex reacted with 1 $\mu$g rat liver RNA (**RL**). The second trace shown is for a positive control (**PC**), in which 30 ng of synthetic probe:miR122a duplex was bound to and eluted from p19 beads. In addition to the positive control samples, performed four negative controls **NC1 to NC4** were performed, as described elsewhere herein. The traces show spikes with current amplitudes typical of dsRNA for samples **PC** and **RL,** whereas no spikes of amplitudes greater than 0.3 nA were present in the negative controls. The open pore current was stable to within 5% throughout the experiments.

To quantify the miRNA concentration, a calibration curve of capture rate vs. concentration was constructed using a synthetic probe : miR122a duplex, indicated by the open markers in Figure 21c. For an unknown miRNA sample, one then counts ~250 current spikes that cross the threshold of $I_o$ - *0.4 nA* (see dashed grey lines in the traces), computes the mean capture rate, and uses the calibration curve to determine the RNA concentration. The PC and RL lines in Figure 21c show how the capture rates translate to RNA concentration for samples **RL** and **PC,** respectively. From the calibration curve, the concentration of the 20-fold diluted miR122a in sample **RL** is 0.7 fmol/ul, translating to an original abundance of 78 $\pm$ 2 pg miR122a/$\mu$g liver RNA in rat liver cells, in close agreement with previous findings (58 - 67 pg miR122a/$\mu$g RNA). In addition, sample **PC** showed a concentration of 5.2 fmol/$\mu$l, indicating that the synthetic probe:miR122a duplex efficiently bound to and eluted from the p19 beads. The negative controls did not show any current spikes that cross the threshold over the measurement time (2 min each), indicating a background that is at least two orders of magnitude lower in spike frequency than sample **RL** (i.e., background noise <7 amol/$\mu$l).

Figure 21d shows the relative error in measured concentration vs. the number of detected molecules. The plot was generated by computing the standard error in the mean capture rates for population subsets of sizes ranging from 100 to 4,000 events. Based on Figs. 5c and d, one can obtain the time required to analyze a sample with a desired accuracy. For 1 fmol miRNA duplex per $\mu$l solution, the capture rate is ~1 molecule/sec, so detection of 250 molecules in ~4 minutes is sufficient to determine miRNA concentration with 93% certainty.

The foregoing demonstrates a process for fabricating uniform, robust and well-defined solid-state membranes that can be manufactured on a full Si wafer, which was used to make solid-state nanopore sensors with the thickness of lipid membranes. Reducing the nanopore thickness improves the signal amplitude from biomolecules, and the use of 3 nm pores in sub-10 nm membranes facilitates electronic discrimination among small nucleic acids. Moreover, nanopores in thin membranes are more easily hydrated than nanopores in thicker membranes, and remain stable over time. Three types of small nucleic acids with different structures were discriminated among with good signal contrast. The systematic study of thin nanopore properties allows development of an electronic detection process for counting individual small RNA molecules, which quantifies miRNA enriched from biological tissue. The inherent ability of these systems to electronically detect single molecules, combined with microfluidic-scale sample volumes (nl) exceeds the detection limits of conventional methods. The systems are capable of simultaneous detection of different molecular species in solution (peptides, miRNA, etc.) by multiplexed read-out of electronic signals from many pores.

**Exemplary Methods**

The substrates for device fabrication were 5x5 mm$^2$ Si chips that have a low-stress silicon nitride (SiN) film deposited on a 5-$\mu$m-thick thermally-grown SiO$_2$ layer, used to reduce the electrical noise. Electron beam lithography was used to write square patterns on the membranes, followed by developing the exposed areas and locally thinning the SiN membrane using an SF$_6$ plasma etcher. After lift-off of the resist and hot piranha cleaning, AFM was used to profile the etch depth (Enviroscope, Veeco). Epi-fluorescence was measured using an upright microscope (Nikon Eclipse 80i) with a Nikon Apo 100x0.95 NA dry objective.

Laser excitation at 488 nm was blocked using a notch filter and detected behind a Chroma 525/50 emission filter using a cooled CCD (Princeton Instruments). Solid-state nanopores were fabricated and analyzed in a JEOL 2010FEG TEM equipped with an annular detector for ADF-STEM. The resulting geometry of nanopores fabricated in solid-state membranes is governed by an interplay between surface tension of the molten SiN and its ablation kinetics. Adjustment of the pore shape by tuning the e-beam fabrication process has been previously reported. In light of a recent report that the TEM beam size influences the nanopore shape, one can use an intense electron beam spot of 1-2 nm diameter to drill the nanopores. All nanopore experiments were carried out using 1M KCl + 1mM EDTA, Tris buffered to pH 8.

An exemplary fluoropolymer cell accommodated volumes of 1-20 $\mu$l and features temperature regulation using a thermoelectric device. The nanopore chip was installed between two buffered electrolyte solutions, each equipped with a Ag/AgCl electrode. After piranha cleaning, each chip was installed in a custom fluoropolymer cell that accommodates volumes as small as 1 $\mu$l. The fraction of pores that yield a steady ion conductance in good agreement with Eqn. 1 using $h_{eff} = h/3$ was ~100% for h < 10 nm, compared to 40-60% for similarly treated pores with h = 25 nm. Pores that exhibited fluctuating currents characterized by high 1/f noise and conductance < 2nS were not reported in Figure 12d and were re-cleaned. When the electrolyte chamber was sealed using a PDMS gasket, or when the cell temperature was reduced to <10°C, the conductance of pores with different thicknesses was stable to within 5% for hours.

For translocation experiments, analyte was added to one of the chambers, and voltage was applied while monitoring current through the pore. Electrical current, measured using an Axopatch 200B amplifier, was digitized at 250 kHz and fed to a computer using custom LabVIEW collection/analysis software. For short nucleic acid analysis one digitally filters the data using a median filter with a rank of 1, in which one finds that events with duration $\geq$24 $\mu$s are undistorted. All DNA samples were purchased from Fermentas (NoLimits®). For the experiments in Figure 12, 4.0$\pm$0.2 nm pores were used, ~1 nM DNA concentrations were placed in one chamber at 21$\pm$0.1°C, and 300 mV was applied to the opposite chamber. The miR122a probe was a 22 nucleotide complementary RNA to miR122a, phosphorylated at the 5' terminus (5'-AACACCAUUGUCACACUCCAUA-3'). The probe:miRNA duplex was enriched from RNA using the protocol described herein. For miRNA determination, ~1 $\mu$l sample was added to the negative chamber of a 3 nm nanopore in a 7 nm thick membrane, and current was recorded vs. time at 500 mV and 0°C. See Figure 21 and associated text. In the first negative control (NC1), 1 $\mu$g of liver RNA was hybridized to a non-specific miRNA probe for miR153 (5'-CACUUUUGUGACUAUGCAA-3'), which is absent in liver. In the second negative control (NC2), no probe was hybridized to 1 $\mu$g of liver RNA. In the third negative control (**NC3**), miR122a probe was hybridized to yeast RNA, which does not contain miR122a. In the fourth negative control (**NC4**), single-stranded miR122a was incubated without liver RNA. In Equation 1 in the geometrical model, one neglects the low SiN surface charge density because experiments were performed at high ionic strengths (1M KCl).

**Fabricating different thickness SiN membranes**

Sub-10 nm thick membranes were fabricated in 5x5 mm$^2$ Si chips that contained a 5-$\mu$m-thick thermal SiO$_2$ oxide underneath a 41 nm thick low-stress SiN layer, deposited by low-pressure chemical vapor deposition (Center for Nanoscale Fabrication, Cornell University). For characterization of the etch rate (see Figure 18c) and for studying DNA transport through 60 nm thick membranes (see Figure 12), a Si wafer that has a 100 nm thick SiN membrane was used. Prior to processing, the thickness of the SiN membrane was measured at different points on the Si wafer using a Rudolph Research AutoEL III Ellipsometer at a wavelength of 632.8 nm and an incidence angle of 70°.

From the ellipsometric parameters, one can obtain for the SiN film optical parameters of $n$ = 2.24, $k$ = 0, as well as a film thickness of 41.5 $\pm$ 0.3 nm. Standard photolithography followed by anisotropic KOH etch was used to produce a freestanding SiN membrane with approximate dimensions ~50x50 $\mu$m$^2$. A subsequent buffered-oxide etch step was then performed to remove the 5 $\mu$m SiO$_2$ layer from the KOH-etched side, in order to obtain freestanding SiN membranes. The SiN membrane was then spun-coated with a PMMA electron beam resist (a 2% solution of 950 kD molecular weight in chlorobenzene, MicroChem Inc.) followed by baking at 180°C for 10 minutes. Electron beam lithography was then used to write a pattern of squares with sides ranging from 250 nm to 5 $\mu$m, using a 50 kV electron beam (Elionix 7500-ELS) and beam dose of 750 $\mu$C/cm$^2$. The irradiated device was developed in 1:3 methyl isobutyl ketone:isopropanol volume ratio for 60 seconds followed by rinsing with isopropanol and drying under a stream of compressed nitrogen gas. Thinning of the exposed SiN areas was accomplished using a Technics PEIIA plasma etcher, using a 50W rf source and 400 mtorr SF$_6$ chamber pressures. The SiN etch rate under these conditions was 1.0 $\pm$ 0.1 nm/s. The resist was finally removed by ~1 h incubation in warm acetone at 65°C, drying under a stream of N$_2$, and then heating at 100°C in hot piranha solution for 10 minutes (made by mixing 1:3 of 30% H$_2$O$_2$ and conc. H$_2$SO$_4$); note that piranha is a strong oxidizer that reacts violently with most organic materials and must be handled with caution. The chips were then washed with water, dried, and stored until use.

**AFM characterization of thinned SiN membranes**

Tapping-mode atomic force microscopy was carried out in order to characterize the process of localized membrane etching. All measurements were performed in ambient air using a Veeco EnviroScope to profile the etch depth and roughness of a given batch of processed chips. TESP Si tips (Veeco) with tip radii of <10 nm were used for all images. In Figure 22, it is shown in panel (a) an AFM image of the pattern shown in Figure 18c, following a 17 nm etch process (the membrane curvature has been subtracted using a polynomial in order to improve visibility of the pattern). The dashed yellow line represents the 3x3 array of 250x250 nm squares that is shown in Figure 18c. In panel (b), three AFM images of 41 nm thick SiN windows in which a 3x3 array of 250 nm squares with a 1 $\mu$m pitch were plasma etched for 17, 33, and 40 seconds. Line profiles through a set of 3 squares can be seen below each image, showing step heights of $17\pm 1$ nm, $33\pm 1$ nm, and for the 40 nm thinning, the image shows a ~100 nm deep triangular profile that resembles the tip shape, indicating that the membrane is completely perforated. The membrane thickness $h$ in the processed region is given by the difference between the initial membrane thickness and the etch depth, i.e., in this case, $h = 41\ nm - etch\ depth$. A membrane robust enough for experiments is a membrane that has been etched for 35 seconds, which yields a membrane thickness of ~6 nm. Membranes with thickness values $h \leq 5$ nm did always not survive the piranha cleaning step.

**Conductance of sub 10 nm thick nanopores as a function of time**

Figure 23 illustrates conductance as a function of time for pores with various diameters $d$ and membrane thicknesses $h$, denoted as $(d, h)$. The buffer used for all measurements was 1M KCl buffered to pH 8. Conductance values are reported in nS based on the current at 300mV and an electrolyte temperature of 21°C.

**Geometric model for $\varDelta I$ of DNA as a function of effective pore thickness**

The values of $\varDelta I$ upon DNA entry were calculated based on *Equation 1* herein, with a modification that takes into account the displaced electrolyte current upon DNA occlusion of the pore. A hydrodynamic diameter for B-form DNA of 2.2 nm was assumed, as it was previously found to explain the observed relative conductance as a function of pore diameter $d$ (see Ref. 41 in the manuscript). The current difference between an open pore $I_o$ (see Eqn. 1 in main text) and a DNA-occluded pore $I_{DNA}$ is expressed as:

$$\text{Eqn. 2:} \qquad \Delta I = I_o - I_{DNA} = I_o - V([\mu_K + (1-S)\mu_{Cl}]n_{KCl}e)\left(\frac{4h_{eff}}{\pi d_{eff}^2} + \frac{1}{d_{eff}}\right)^{-1}$$

where the two added parameters are $d_{eff}$, the effective pore diameter of the DNA-occluded pore (calculated from a circle of an equivalent area that is available for KCl transport in the case of the DNA occluded pore case), and $S$, the fraction of excluded Cl$^-$ ions in the DNA-occluded pore. This exclusion of Cl$^-$ ions was modelled as a coefficient from 0-1 that reduces the mobility of Cl$^-$ ions (this is equivalent to a reduced Cl$^-$ concentration). The rational for chloride exclusion is based on previous results that showed an increase in $\varDelta I$ with DNA length for a length >1,200 bp, which may be attributable to a decreased effective concentration of anions (e.g., Cl$^-$) near the pore during translocation, due to electrostatic repulsion by the DNA coil. The dashed line in Figure 12d is a best fit to $\varDelta I$ for a pore diameter $d = 4$ nm as a function of the effective thickness $h_{eff}$, which yields values of $d_{eff} = 2.83$ nm and S = 0.2 (i.e., 20% exclusion of Cl$^-$).

**Scatter plots of $\Delta$I vs. transport time of 3 kbp DNA for two pore thicknesses**

Figure 24 shows a scatter plot of the mean current amplitude of each molecule ($\Box I$) and the total transport time for 3 kbp dsDNA through 4 nm pores as a function of membrane thickness ($h$). Thinning the membrane has two observable consequences, one is increased $\Box I$, which facilitates detection of the DNA, and the second is a minor decrease in transport times. Overall, transport times were ~30% smaller when reducing $h$ from 60 to 6 nm, going from 0.95 ms to 0.72 ms. The plot on the right of Figure 24 shows transport time distributions for two membrane thicknesses. Mean transport times are determined by fitting the distributions to exponentially decaying distributions.

**Translocations of 10 bp DNA through a 3 nm diameter pore in a 7 nm thick membrane**

Figure 25 shows a set of 10 bp translocations through a 3 nm diameter pore in a 7 nm thick membrane under 500 mV applied voltage, at a temperature of 0°C. The events were concatenated by pasting together current spikes that include 2 ms current data before and after each spike. In contrast to events with the 25 bp DNA fragment, one observes many deep and long events for the 10 bp sample, which was observed for many different 3 nm diameter pores. This may be attributed to sideways jamming of the molecule at the pore entrance, which would cause the DNA to stall for a relatively long time (milliseconds). The contour length of the 10 bp DNA is 3.5 nm, slightly higher than the pore diameter.

An all-point histogram is shown to the right of the concatenated trace. The difference in amplitude between the first blockade peak and the open pore peak is 0.6 nA, similar to that of the 25 bp DNA sample (see Figure 20). The second peak (1.1 nA) may be due to entry of two molecules simultaneously or sideways entry of the molecule into the pore. The traces for 25 bp DNA and the 22 bp RNA samples in Figure 20a show that such frequent blocking of the pore does not occur in double-stranded nucleic acids with contour lengths of at least 7 nm.

**Discrimination among 25 bp DNA, 22 bp RNA, and 76-nucleotide tRNA**

Figure 26 provides more detail about using a 3 nm diameter pore in a 7 nm thick membrane to discriminate among small nucleic acids of similar size, namely, 25 bp DNA (molecular weight = 15kD), 22 bp RNA (molecular weight = 15 kD), and 76-nucleotide tRNA (molecular weight = 25 kD). Figure 26a illustrates scatter plots of current amplitude ($I$) vs. transport time for the three molecules under the same measurement conditions (0°C, 500 mV, 3 nm diameter pore in a 7 nm thick SiN membrane). The dashed ovals represent regions containing >85% of detectable events for each molecule type. Figure 26b illustrates transport-time distributions for >1,000 events of each molecule type. For the 25 bp DNA and 22 bp RNA, >90% of the data fits a single exponential decay, with timescales of 20 $\mu$s and 50 $\mu$s, respectively.

For tRNA, the data fits a two-exponential function, with timescales 80 $\mu$s and 1.04 ms (see inset of panel b for expanded view of the transport-time histogram for tRNA). While the short timescale may be due to collisions and fast translocations of the bulky tRNA molecule, the long timescale, in which the majority of events fall in, suggests that the tRNA must deform from its bent equilibrium structure in order to traverse the 3 nm diameter pore, a process that may stall transport, leading to longer dwell times and a broader dwell time distribution. Representative concatenated translocation events (Figure 26c) in the dwell time range of 12 - 228 $\mu$s for 25 bp DNA (c) and 22 bp RNA. In Figure 26d, events for the tRNA molecule are not shown here, because their duration is clearly longer, as seen in Figure 20a. The corresponding transport times are noted below each event. These traces are shown in order to illustrate that the current amplitude for 25 bp DNA and 22 bp RNA are distinctly different, easily seen for events with duration longer than 30 $\mu$s. These results suggest that the different amplitude spikes are a result of the different molecular properties between the three nucleic acids, rather than an artifact of fast dwell times close to the detection limit that would distort the current amplitudes.

A more quantitative analysis of the nanopore's ability to discriminate 25-bp DNA from 22-bp RNA based on current amplitudes is shown in Figure 27. First, in order to test the response of the system to short pulses, a simulation was performed of ideal square current pulses with different durations and current amplitudes in the range 8 - 48 $\mu$s (see APPENDIX A at the end of this document). The simulated events indicate that all events with durations < 20 $\mu$s are attenuated in terms of their amplitudes. For example, for pulse widths of 8 $\mu$s, the mean attenuation factor is 20%. That is, events with 500 pA and 750 pA amplitudes are detected as 400 pA and 600 pA amplitudes, respectively.

Next, is shown the effect of a rank 1 median filter on the real DNA and RNA data collected for the scatter plots shown in Figure 26. Shown in the figure are $\Delta I$ distributions of for all the events in the scatter plots for 25-bp DNA and 22-bp RNA . In this series of histograms, one takes subsets of the dataset that contain events with duration equal to or longer than the indicated value in each plot.

Also indicated in the plots are % certainty values for the discrimination between DNA and RNA, calculated from the fraction of RNA events that lie above the $I_{Thres}$= 675 pA threshold (shown as dashed line). The threshold was chosen because <0.1% DNA events were found with larger $\Delta I$ values. For example, including only events ≥8 $\mu$s results in a certainty of 83.3%, because 16.7% of the RNA events have amplitudes that are lower than $I_{Thres}$. In other words, looking at all of the data in the range ≥8 $\mu$s, one finds that 16.7% of the RNA molecules and mixed with the DNA population, and therefore cannot be distinguished from DNA events. Now, when one looks to data ≥12 microseconds, one finds that 91.3% of the RNA events lie above the threshold, resulting in only 8.7% error in discrimination. This certainty in discrimination improves to >97% when only events ≥16 $\mu$s are included. One notes that events with durations ≥16 $\mu$s constitute 75% and 73% of the total detected events for DNA and RNA, respectively. Finally, one notes that differences in duration between DNA and RNA cannot explain the differences in current amplitudes, because events longer than 36 $\mu$s, which represent about one half of the datapoints, have a clear plateau at the mean current amplitude value, unequivocally showing that DNA and RNA provide distinguishable current amplitude levels.

The bandwidth-limited data acquisition results in asymmetric shapes for the current amplitude distributions, which exhibit "bellies" or "shoulders" at 0.4 nA and 0.6 nA for DNA (left arrow) and RNA (right arrow), respectively. These "bellies", which deviate from Gaussian shapes (see curves), disappear completely for events with durations of ≥16 $\mu$s, and the distribution shapes are Gaussian thereafter. From the simulations shown in Appendix A, this asymmetry may be a result of attenuation by filter.

However, despite the signal attenuation, one still can discriminate among short DNA and RNA, provided that one chooses a suitable threshold amplitude for discrimination that considers the event duration. To illustrate this, shown are $\Delta I$ histograms for DNA and RNA events where in each plot are analyzed slices of the data that select all events with an indicated duration, in the range of 8-36 $\square$s. In each plot are drawn dashed lines for $\Delta I_{Thres}$ = 675pA as a guide to the eye. In Figure 28, below all the distributions are shown mean $\Delta I$ values as a function of event duration (the error bars show one standard deviation in each direction). As seen in these plots, the current amplitude signals for *both* RNA and DNA are distorted towards lower $\Delta I$ values for events in the range 8-16 $\square$s, after which there is no apparent shift in the position of the distributions. The magnitude of this distortion for short events is up to 150 pA for the 8 $\square$s events (or 22% of the $\Delta I$ value), in good agreement with the simulated data. The attenuation factor appears robust as a function of event duration, and therefore one can compensate for attenuated data by adapting the threshold (see lower arched, dashed line in Figure 28 that connects up to straight dashed line at duration = 22 microseconds), in order discriminate DNA from RNA in this regime with >90% confidence.

## Continuous traces for 25 bp DNA through a 3 nm pore for different voltages

Figure 29 shows 2-second current traces under different applied voltages of a 25 bp DNA sample analyzed using a 3 nm diameter pore fabricated in a 7 nm thick membrane (data taken at a temperature of 0°C). Pores are not, of course, limited to 3 nm diameters, as pores can have diameters of 1 nm, 5 nm, 10 nm, 25 nm, 50 nm, 100 nm, and of intermediate valuestherebetween. The analyte chamber contains 25 bp DNA at a concentration of 81 fmol/$\mu$l, and positive voltage is applied to the second chamber in order to drive DNA to the other side. The inset trace in each plot is a zoomed-in 7 ms view, with the same current amplitude scale as the y-axis. On the right-hand side of the figure, all-point current histograms are shown for each trace. Increasing the voltage greatly increases the capture rate, as seen by the increasing amount of deep events. The shallow events for the low voltage traces are presumably collisions of the DNA with the pore. The fraction of these collisions decreases with increasing voltages, in line with a voltage-activated barrier for threading (see manuscript text).

## Continuous time traces for 25 bp DNA at different concentrations

Figure 30 shows current vs. time traces for a 3 nm diameter pore at a measured at a voltage of 500 mV and a temperature of 0°C, when different concentrations of DNA were added to the pore (expressed as fmol/$\mu$l solution). While the open pore current remains similar for different concentrations of DNA (~2.5 nA), the number of spikes increases as a function of the concentration. The rate of events shown in Figure 12d were derived from the time-delay distributions ($\square$t) between two successive events (see plot below the traces), which fits a first arrival time distribution function $P_{capture}$ = $Aexp(-Rate*t)$, where the slope of the exponent (i.e., *Rate*) is the mean capture rate in s$^{-1}$.

## Details of p19-based miRNA enrichment from cellular RNA extracts

The p19 protein binds tightly to double-stranded RNA that is 19 to 22 basepairs in length. There is no binding to single stranded RNA. This tight, selective binding of p19 to dsRNAs allows the enrichment from cellular RNA of probe-hybridized miRNAs that have a very low abundance. For example, miR153, a miRNA with very low abundance compared to other miRNAs, has been enriched by over 100,000-fold from cellular RNA (see Ref 54 in the manuscript).

The protocol of miRNA enrichment proceeds as follows: A synthetic 22 nucleotide RNA oligo probe of sequence 5'-AACACCAUUGUCACAC-UCCAUA-3' (Integrated DNA technologies, Inc.) complementary to miR122a was first phosphorylated at the 5' end using T4 polynucleotide kinase (New England Biolabs, Ipswich, MA). The miR122a-specific probe was then added to PCR tubes containing the different RNA samples (rat liver RNA, positive control, and the four negative controls) in IX p19 binding buffer (20 mM Tris-HCl, pH 7.0, 1mM EDTA, 1 mM tris (2-carboxyethyl) phosphine, 100 mM NaCl, 0.02% Tween-20) in a total volume of 10 $\mu$l. Hybridizations were carried out in a thermal cycler programmed to 75°C for 5 min, followed by 52°C for 5 hours. Each 10 $\mu$l of hybridization reaction was incubated with 10 $\mu$l of p19 beads suspended in IX p19 binding buffer, 10 units of murine RNase inhibitor (NEB), and 1 mg of BSA in a total volume of 20 $\mu$l. The binding reaction was incubated by shaking for 1-2 h at RT in an Orbis shaker (MarketLab, Caledonia, MI, USA). Using a magnetic rock (NEB), the unbound RNA was removed by washing 6 times in 600 $\mu$l of 1X p19 wash buffer (20 mM Tris-HCl, pH 7.0, 1 mM EDTA, 100 mM NaCl). For each wash, the beads were shaken for 5 min. at 37°C on a heated shaker. After the third wash, the washing temperature was increased to 42°C to remove all of the non-specific unbound RNA. The probe:miR122a duplex was eluted from the p19 beads into 20 $\mu$l of IX p19 elution buffer (20 mM Tris-HCl adusted to pH 7.0, 100 mM NaCl, 1 mM EDTA and 0.5% SDS) by shaking for 20 min at 37°C. SDS was removed by adding 16 $\mu$l of 4 M KCl to the 160 $\mu$l eluate and cooling to 4°C. After centrifugation for 15 min. at 14,000 rpm in a microfuge, the solution was carefully decanted to a new tube to remove the white SDS pellet. The isolated miRNA were diluted into KCl solutions such that the total KCl concentration was 1M, and the solution was added to a 3 nm diameter nanopore in a 7 nm thick membrane for detection, which was carried out for all samples at 0°C and 500 mV.

The sequence of experiments that led to the data for Figure 21 is as follows: A calibration curve for different 22 bp duplex RNA concentrations was first performed using the synthetic probe:miR122a duplex, after which the negative controls **NC1-NC4** were tested, and finally, samples **RL** and **PC** were tested. Between each sample, the chamber was rinsed 10 times by removing the chamber contents, adding 20 $\mu$l of fresh buffer, mixing the chamber contents using a pipette, and repeating the process. After each cleaning procedure, a 20 second trace was collected to verify that the baseline current was within 5% of the open pore current value, and that no detectable events are present. During data acquisition for the negative controls, evidence that the nanopore is active comes from the fact that occasionally shallow events occurred (<0.3 nA), which may be tied to trace amounts of single-stranded RNA (e.g., the hybridization probe) present in the p19-treated samples.

**Continuous time traces of different concentrations of 22 bp RNA**

Figure 31 shows continuous time traces for a 3 nm diameter pore measured at a voltage of 500 mV and a temperature of 0°C, when different concentrations of RNA were added to the pore (expressed as fmol/$\mu$l solution). The calibration curve in Figure 21c was constructed by calculating for each trace the mean capture rate as a function of the RNA concentration.

**Response of system to artificial short current pulses**

In order to test the system's response to very fast translocation events, the response of the amplifier tested to synthetic current pulses in the range 8 - 48 $\square$s was tested. The scheme for generating these pulses is shown in Figure 32: A 2 MHz square wave generator with asymmetric pulse capabilities (TENMA Jupiter 2010) was fed into the compensated RC circuitry shown in Figure 32. The circuitry converts the voltage signal of the generator into a current pulse train with specified durations and amplitudes, adjusted manually by reading the function generator's output using an oscilloscope. Next, were generated current pulses of durations in the range 8 - 48 microseconds, fed the signal into the amplifier's headstage, then digitized the output at a sampling rate of 250 kHz using a DAQ card and median filtered the data using a rank of 1. Combined with the 100 kHz bandwidth of the Axopatch 200B patch clamp amplifier, which is set by its internal 4-pole Bessel filter, a rank 1 median filter behaves as a low-pass filter with a corner frequency of $f_c$=37.8 Khz at 250 kHz sampling rate. The roll-off of this median filter is shallower than commercial low-pass filters.

Representative traces are shown for pulses of the indicated durations and two amplitudes, *delta I*= 500 pA (left column of plots) and 750 pA (right column of plots), which model events from the short DNA and RNA molecules, respectively. A plot of the mean delta *I* values vs. pulse duration shows that events of duration $\leq$20 microseconds are attenuated. However, the attenuation factor is similar for the 500 pA and the 750 pA pulses: for the 8 microsecond pulses that are detected as a single sample point, the ***peak*** amplitude was attenuated by 14% for both pulse amplitudes.

**Exemplary Embodiments**

One exemplary embodiment of the disclosed devices is shown in Figure 33. The device will be described here illustrating its use in detecting miRNA, but the device may be used for detecting other analytes, such as DNA and RNA.

In some embodiments, the devices may include a Si-chip. Such chips may have an area of only a few mm$^2$. For example, one may use a 5 x 5 mm$^2$ device, with a transparent SiN window that is 100x100 $\mu$m$^2$. The devices may integrate the disclosed nanopore/electrode devices with fluidics such that analyte isolation (e.g., p19-based miRNA isolation) can be performed in real time.

In some embodiments (illustrated by Figure 21a), the device accepts an input sample (such as cell contents, including cellular RNA), hybridizes the sample to a probe (e.g., a miRNA probe), immobilizes the probe:analyte duplex (e.g., onto protein p19). The user may then wash, elute, and detect the analyte (miRNA) concentration. These devices thus enable multiplexed delivery and detection of miRNAs using a series of nanopores equipped with electrodes.

The signals (such as changes in electrical current) may be read using independent electrodes that are disposed proximal to each nanopore. The devices are suitably constructed such that nanopores are individually monitored or addressed by electrodes. In this way, the user may assign different analytes to different nanopores, enabling simultaneous, parallel analysis of multiple analytes.

By analyzing the current signal as a function of time, the concentration of each miRNA in each channel is quantified, which may be achieved by comparing the signal (or passage events per time) to a calibration curve. Exemplary electrodes are described in United States Published Applications US 2010/0142259 ("Nanogaps: Methods and Devices Containing Same") and US 2010/0009134 ("Beam Ablation Lithography').

In the exemplary embodiment shown in Figure 33, a device includes an input chamber. The user may introduce sample material (e.g., the contents of a lysed cell or cells) into this input chamber. The chamber may itself be a lysis chamber, configured to apply or receive lysis reagents, heat, sonication, pressure, and the like to a cell or other sample placed within the chamber. Lysing techniques and reagents are known in the art, and suitable lysing techniques will be known to the user of ordinary skill in the art. The lysing may be accomplished, for example, on a porous membrane under pressure. Alternatively, lysing may be accomplished chemically. In some embodiments, cell contents may be introduced directly into the chamber.

The input chamber may be configured or disposed such that it is in fluid communication with one or more nanopores according to the claimed invention. The fluid communication may be accomplished by having tubes, conduits, channels, or other pathways between the input chamber and the nanopores. The input chamber may have multiple outlets, where each outlet is connected to one or more channels. Alternatively, the input chamber may include or be connected to a manifold, which manifold in turn distributes sample material to the channels. The manifold may be constructed so as to admit material into only a single channel or into multiple channels. The devices suitably include a device that applies a gradient (e.g., fluid pressure, voltage, magnetic) to transport sample or analyte from one location in the device to another.

The exemplary embodiment of Figure 33 illustrates channels connecting the chamber to the nanopores. The chamber may suitably have an internal volume of 0.1 - 100 ml, or 1 - 10 ml, or even about 5 ml, depending on the needs of the user; although larger and smaller volumes are also suitable. The chambers are suitably constructed from polymers, silicon, and from other materials known and used in the fluidics field.

The devices may be constructed such that the capture material, nanopores, channels, or all of the foregoing, are disposed on a chip or other device that is then connected to a sample source or input chamber. In other embodiments, the chamber, nanopores, channels, electrodes and capture materials are all disposed on a single, integrated device, as shown in Figure 33. The components may be configured as part of a system, where a base or other unit accepts chips having sample containers, capture materials, nanopores, and electrodes in a snap-in or slide-in configuration. The base may include electrode that contact electrical contacts in the chips so as to apply a gradient or to address individual pores. The system may also include pumps, syringes, and/or other means that deliver fluids and samples to the input chambers, channels, pores, or other components.

The devices may be constructed such that each channel has a length of from 0.1 mm to 1 cm or even 10 cm or more. Channels may have internal cross-sectional dimensions of from 0.1 mm to 1 cm, or 1 mm to 100 mm, or even about 5 mm to 10 mm. The channels may be made from a polymer, silicon, an oxide, or other materials used in the fluidics field and known to those of skill in the art.

In some embodiments, one or more channels may include a material that binds to a particular analyte or analytes. For example, in the case of miRNA, the capture material may be p19 (e.g., New England Biolabs, www.neb.com) protein or other material that binds specifically to miRNA. While the p19 protein is considered especially suitable, other proteins that resemble p19 in structure or in function are suitable, as well as mutants derived from the native viral sequence. The material may be cellulose, a Drosha-DGCR8 complex, the PAZ domain on the *C. elegans* genome, and the like. The capture material may be present on a bead, porous support, or other structure, as shown in Figure 21. Beads are considered especially suitable, but are not necessary to the disclosed devices.

In this way, the user may transport cell contents to the capture materials so as to isolate an analyte (e.g., miRNA), if present, in the sample. Once the target binds to the capture material, the user may flush the channel (and capture material) to remove any unwanted materials.

In certain embodiments, the user may apply an analyte-specific probe to the analyte. Suitable probes include multi-nucleotide probes or even proteins; probes that specifically bind to the analyte of interest are considered especially suitable. The probe may be labeled with a fluorophore or phosphor. A probe may also be labeled magnetically or radioactively. In some embodiments, the analyte (e.g., miRNA) and probe are mixed together, and the capture material binds to the analyte-probe duplexes. In other case, a capture material - such as a bead or a porous support - has bound to it probes that are specific to a particular analyte. For example, a bead (or other support, such as a monolith or a strip) may be decorated with probes that are complementary to a nucleotide sequence on miRNA1. The sample is contacted to the bead, and miRNA1 in the sample - if present - binds to the probes on the bead. Excess sample may be washed away, and the bound miRNA1 may be eluted (in duplex miRNA1 :probe form or as miRNA1 alone) and detected at a nanopore. The capture material may also be a microarray, which micro array includes spots or regions that bind specifically to only a particular analyte. For example, such a region on a microarray might include oligonucleotides or even proteins that are specifically complementary to an analyte of interest. Once the analyte binds and excess sample is washed away, the bound analyte is suitably eluted and then detected by a nanopore.

In this way, a device may detect multiple analytes, and may even do so in real time. In one embodiment, the sample chamber is in fluid communication with a first capture material (binding specifically to miRNA1) and a second capture material (binding specifically to miRNA2). Each of these capture materials may be disposed within its own channel, which channel is in turn in fluid communication with its own nanopore. The sample is contacted to the first and second capture materials, which materials bind specifically (respectively) to miRNA1 and miRNA2, if present. The user may then wash away excess sample. The bound miRNA1 and miRNA2 are then eluted into the channels associated with the respective capture materials and are detected by the nanopores associated with those channels, where the user detects the concentration (e.g., in the form of miRNA passages through the nanopore per unit time) of each miRNA.

The probe is suitably designed or selected so as to be complementary to only a particular target. By applying multiple probes to multiple capture materials, the user can simultaneously label multiple analytes. For example, capture material is disposed in channels 1 and 2 of a given device. The user then contacts the bound miRNA in channel 1 with a probe that is complementary only to miRNA1, and contacts the bound miRNA in channel 2 with a probe that is complementary only to miRNA2.

The user elutes any bound miRNA from the capture material (e.g., by application of heat, solvent, reagent, and the like), and the eluted miRNA then travels down the channels to a nanopore or nanopores. Once the eluted miRNA arrives at the nanopores, electrodes monitor the number of miRNA probe-target duplex passage events through the nanopore. In embodiments where the analyte is labeled with a fluorescent, magnetic, or radioactive probe, the nanopores may be used to count the number of visual, magnetic, or radioactive passage events per unit time. The number of passage events may be correlated to the concentration of the analyte; this may be done with the assistance of a calibration curve, as shown in Figure 21c.

Target material of interest may be bound to a probe for enrichment, or may be in its natural form (i.e., not bound to a probe). The probe may be selected such that the probe binds specifically to the analyte of interest. The probe may also bind to the capture material; in this way, only analyte bound to a probe binds to the capture material, and unbound analyte may be washed away. In some embodiments, the probe is not labeled, and gives rise to a sample:probe duplex than is then isolated and detected by passage through a nanopore. (Nanopores are suitably configured according to any of the nanopore devices and membranes described herein.)

Each channel in a device may be separately addressable; the nanopores are also suitably individually addressable. For example, the first channel in a device may be used to detect the concentration of miRNA1. This may be accomplished by eluting into the nanopore only analyte material that has been exposed to a probe complementary to miRNA1. If duplexes between that material and the probe are present, the user will observe passage events of the duplexes through the nanopore. If no such duplexes are present - e.g., because miRNA1 was not present in the cell sample and the miRNA1 probe consequently had nothing to bind to and was washed away - then the user will observe little to no passage events at the nanopore. The user may create a multiplexed device - as shown in Figure 33 - that includes multiple channels for application of multiple probes, which in turn allows for detection of multiple analytes. A device may be constructed having two, three, or more channels dedicated to a single analyte (e.g., three channels configured to detect miRNA1) so as to perform multiple, simultaneous detections for a single analyte.

The devices may include nanopores and membranes according to any of embodiment disclosed in this application. For example, the devices may include a first capture material that binds specifically to a first molecule; a first membrane having a thickness in the range of from about 5 nm to about 100 nm; and a second membrane disposed adjacent the first membrane, the second membrane having a thickness in the range of sub-nm thickness up to about 20 nm (and all values therebetween), with the second membrane having at least one pore extending therethrough, the pore having a cross-sectional dimension in the range of from about 1 nm to about 100 nm, and the first membrane having a cavity formed thereon, the cavity being in register with at least one pore of the second membrane, the first pore being in fluid communication with the first capture material; a device configured to apply a gradient across the pore; and a detector configured to detect a signal related to passage of a molecule through the first pore.

In another embodiment, the device includes a first capture material configured to bind preferentially to a first molecule; a membrane having a thickness in the range of from about 20 nm to about 100 nm, and the membrane having a thinned region, the thinned region having a thickness in the range of from about 0.1 nm to about 20 nm, and a first pore extending through the thinned region, the first pore being in fluid communication with the capture material; and a detector configured to detect a signal related to passage of the first molecule through the first pore.

While the foregoing examples pertain to miRNAs, the described devices and methods may be used to detect analytes other than miRNA. For example, the devices may be used to detect DNA, RNA, tRNA, mRNA, and the like.

Also provided herein are methods of obtaining sequence or structure information. In these methods, the user may translocate an analyte through a nanopore (described elsewhere herein), and detect a signal related to the structure of the analyte. The signal may be an electrical signal, an optical signal, a magnetic signal, and the like. The signal is suitably related to a structural feature of the analyte. For example, a user may contact a DNA molecule with a labeled probe that bind only to a specific sequence of nucleic acids, e.g., ATTCG. The user may then translocate the sample through a nanopore and detect - if present - a signal (e.g., an optical signal from a fluorescent probe) related to passage of the labeled probe through the nanopore. The signal may, as described elsewhere herein, be correlated to the concentration of the target analyte.

The presence of the signal will indicate that the sequence to which the probe is complementary is present on the analyte. The user may perform sequencing by contacting segments of a target analyte (e.g., segments that are generated by restriction enzyme or enzymes) with labeled, sequence specific probes, and then detecting the presence - or absence - of signals when the segments are translocated through the nanopore.

Alternatively, the user may apply restriction enzymes with known binding sequences to an analyte and detect the presence - or absence - of segments cleaved from the analyte by the restriction enzyme. In this way, if a segment is detected, the user will know that the analyte from which that segment was removed included the sequence of nucleic acids to which the restriction enzyme bound.

The foregoing description and attached figures are illustrative only, and do not limit the scope of the present disclosure or claims. Variations on the foregoing description are also within the scope of the present disclosure. A number of references are provided below.

**Claims**

1.    An analysis device, comprising:

a membrane having a thickness in the range of from 0.2 nm to 100 nm,
wherein the membrane having a thickness in the range of from 0.2 nm to 100 nm has a locally thinned area;
at least one pore extending through the locally thinned area of the membrane, wherein the at least one pore has a characteristic cross-sectional dimension in the range of from 1 nm to 1000 nm; and
a supporting layer adjacent to the membrane.

2.    The analysis device of claim 1, wherein the membrane comprises a nitride or an oxide.

3.    The analysis device of claim 2, wherein the nitride comprises silicon nitride, boron nitride, titanium nitride or gallium nitride.

4.    The analysis device of claim 2, wherein the oxide comprises silicon oxide, hafnium oxide, titanium oxide or aluminium oxide.

5.    The analysis device of claim 1, wherein the pore has a cross-sectional dimension in the range of from 1 nm to 200 nm.

6.    The analysis device of claim 1, wherein the supporting layer adjacent to the membrane has a thickness in the range of from 1 micron to 2000 microns.

7.    The analysis device of claim 1, further comprising a dielectric layer disposed adjacent to the membrane.

8.    The analysis device of claim 7 where the dielectric layer material comprises silicon oxide, aluminium oxide or silicon nitride.

9.    The analysis device of claim 1, further comprising a device capable of applying a gradient across the pore.

10.   The analysis device of claim 1, further comprising a monitoring device capable of detecting a signal related to passage of a macromolecule across the pore.

11.   The analysis device of claim 10, further comprising a device capable of comparing the signal related to passage of a macromolecule across the pore to a signal evolved from passage of a macromolecule of known structure across the pore.

12.   A method of analysing a macromolecule, comprising:

translocating at least a portion of a macromolecule through a pore disposed in a thinned region of a membrane, the thinned region of the membrane having a thickness in the range of from 0.1 nm to 20 nm;
monitoring a signal related to the translocation of the macromolecule through the pore; and
correlating the signal to a structural property of the macromolecule.

13.   The method of claim 12, wherein the macromolecule comprises a nucleic acid.

14.   A method of fabricating an analysis device, comprising:

removing at least a portion of a resist material disposed adjacent to a membrane so as to expose a target region of the membrane;
etching at least a portion of the target region of the membrane material so as to reduce the thickness of the membrane material within the target region; and

forming a pore that extends through the thinned target region of the membrane material.

**Patentansprüche**

1. Analysevorrichtung, umfassend:

   eine Membran mit einer Dicke in dem Bereich von 0,2 nm bis 100 nm,
   wobei die Membran, die eine Dicke in dem Bereich von 0,2 nm bis 100 nm aufweist, einen lokal verdünnten Bereich aufweist;
   wenigstens eine Pore, die durch den lokal verdünnten Bereich der Membran verläuft, wobei die wenigstens eine Pore eine charakteristische Querschnittsabmessung in dem Bereich von 1 nm bis 1000 nm aufweist; und
   eine Trägerschicht benachbart zu der Membran.

2. Analysevorrichtung gemäß Anspruch 1, wobei die Membran ein Nitrid oder ein Oxid umfasst.

3. Analysevorrichtung gemäß Anspruch 2, wobei das Nitrid Siliciumnitrid, Bornitrid, Titannitrid oder Galliumnitrid umfasst.

4. Analysevorrichtung gemäß Anspruch 2, wobei das Oxid Siliciumoxid, Hafniumoxid, Titanoxid oder Aluminiumoxid umfasst.

5. Analysevorrichtung gemäß Anspruch 1, wobei die Pore eine Querschnittsabmessung in dem Bereich von 1 nm bis 200 nm aufweist.

6. Analysevorrichtung gemäß Anspruch 1, wobei die Trägerschicht benachbart zu der Membran eine Dicke in dem Bereich von 1 Mikrometer bis 2000 Mikrometer aufweist.

7. Analysevorrichtung gemäß Anspruch 1, ferner umfassend eine dielektrische Schicht, die benachbart zu der Membran angeordnet ist.

8. Analysevorrichtung gemäß Anspruch 7, wobei das Material der dielektrischen Schicht Siliciumoxid, Aluminiumoxid oder Siliciumnitrid umfasst.

9. Analysevorrichtung gemäß Anspruch 1, ferner umfassend eine Vorrichtung, die fähig ist, einen Gradienten über die Pore anzulegen.

10. Analysevorrichtung gemäß Anspruch 1, ferner umfassend eine Überwachungsvorrichtung, die fähig ist, ein Signal zu erfassen, das mit dem Durchtreten eines Makromoleküls durch die Pore in Beziehung steht.

11. Analysevorrichtung gemäß Anspruch 10, ferner umfassend eine Vorrichtung, die fähig ist, das Signal, das mit dem Durchtreten eines Makromoleküls durch die Pore in Beziehung steht, mit einem Signal aus dem Durchtreten eines Makromoleküls mit bekannter Struktur durch die Pore zu vergleichen.

12. Verfahren zum Analysieren eines Makromoleküls, umfassend:

    Translozieren wenigstens eines Teils eines Makromoleküls durch eine Pore, die in einem verdünnten Bereich einer Membran angeordnet ist, wobei der verdünnte Bereich der Membran eine Dicke in dem Bereich von 0,1 nm bis 20 nm aufweist;
    Überwachen eines Signals, das mit der Translokation des Makromoleküls durch die Pore in Beziehung steht; und
    Korrelieren des Signals mit einer Struktureigenschaft des Makromoleküls.

13. Verfahren gemäß Anspruch 12, wobei das Makromolekül eine Nukleinsäure umfasst.

14. Verfahren zur Herstellung einer Analysevorrichtung, umfassend:

    Entfernen wenigstens eines Teils eines Resistmaterials, das benachbart zu einer Membran angeordnet ist, um einen Zielbereich der Membran freizulegen;

Ätzen wenigstens eines Teils des Zielbereichs des Membranmaterials, um die Dicke des Membranmaterials innerhalb des Zielbereichs zu verringern; und

Bilden einer Pore, die durch den verdünnten Zielbereich des Membranmaterials verläuft.

## Revendications

1. Dispositif d'analyse comprenant :

une membrane d'épaisseur comprise entre 0,2 nm et 100 nm,

où la membrane d'épaisseur comprise entre 0,2 nm et 100 nm présente une zone localement étrécie ;

au moins un pore s'étendant à travers la zone localement étrécie de la membrane, où l'au moins un pore présente une dimension de section transversale caractéristique comprise dans l'intervalle allant de 1 nm à 1000 nm ; et

une couche de support adjacente à la membrane.

2. Dispositif d'analyse selon la revendication 1, où la membrane comprend un nitrure ou un oxyde.

3. Dispositif d'analyse selon la revendication 2, où le nitrure comprend du nitrure de silicium, du nitrure de bore, du nitrure de titane ou du nitrure de gallium.

4. Dispositif d'analyse selon la revendication 2, où l'oxyde comprend de l'oxyde de silicium, de l'oxyde d'hafnium, de l'oxyde de titane ou de l'oxyde d'aluminium.

5. Dispositif d'analyse selon la revendication 1, où le pore présente une dimension de section transversale dans l'intervalle compris entre 1 nm et 200 nm.

6. Dispositif d'analyse selon la revendication 1, où la couche de support adjacente à la membrane présente une épaisseur comprise dans l'intervalle allant de 1 micron à 2000 microns.

7. Dispositif d'analyse selon la revendication 1, comprenant en outre une couche diélectrique disposée de façon adjacente à membrane.

8. Dispositif d'analyse selon la revendication 7, où le matériau de la couche diélectrique comprend de l'oxyde de silicium, de l'oxyde d'aluminium ou du nitrure de silicium.

9. Dispositif d'analyse selon la revendication 1, comprenant en outre un dispositif capable d'appliquer un gradient sur le pore.

10. Dispositif d'analyse selon la revendication 1, comprenant en outre un dispositif de suivi capable de détecter un signal lié au passage d'une macromolécule à travers le pore.

11. Dispositif d'analyse selon la revendication 10, comprenant en outre un dispositif capable de comparer le signal lié au passage d'une macromolécule à travers le pore à un signal provoqué par le passage d'une macromolécule d'une structure connue à travers le pore.

12. Méthode d'analyse d'une macromolécule, comprenant :

la translocation d'au moins une portion d'une macromolécule à travers un pore disposé dans une région étrécie d'une membrane, la région étrécie de la membrane ayant une épaisseur comprise dans l'intervalle allant de 0,1 nm à 20 nm ;

le suivi d'un signal lié à la translocation de la macromolécule à travers le pore ; et

la corrélation du signal à une propriété structurelle de la macromolécule.

13. Méthode selon la revendication 12, où la macromolécule comprend un acide nucléique.

14. Méthode de fabrication d'un dispositif d'analyse, comprenant :

l'élimination d'au moins une portion d'un matériau de résist adjacent à une membrane de sorte à exposer une région cible de la membrane ;

la gravure d'au moins une portion de la région cible du matériau de membrane de sorte à réduire l'épaisseur du matériau de membrane à l'intérieur de la région cible ; et

la formation d'un pore qui s'étend à travers la région cible étrécie du matériau de membrane.

Figure 1:

Figure 2:

Figure 3:

Figure 4:

## Figure 5:

Electron beam lithography, development

Figure 6:

## Figure 7:

Resist Removal

Figure 8:

Nano-pore Drilling

Figure 9:

Figure 10

Figure 11

25 bp dsDNA

200 pA

1 ms

Figure 12a

Figure 12b-c

Figure 12d-e

Figure 13

Figure 14

Figure 15

Electron beam lithography, development

Figure 16

Plasma etching

Figure 17

Nano-pore Drilling

Figure 18a

Figure 18b-c

Figure 18d

Figure 19a-b

Figure 20a

Figure 20b-c

Figure 21a

a

Other RNAs
Probe
miRNA

p19

Probe : miRNA
duplex

← 3 nm →

EP 2 537 026 B1

53

Figure 21b

Figure 21c-d

Figure 22

Figure 23

Figure 24

Figure 25

Figure 26a-b

Figure 26c

d. 22 bp RNA translocation events

Figure 27

Figure 28

EP 2 537 026 B1

Figure 29

65

Figure 30

Figure 31

Figure 32

Figure 33

Figure 34

# (layer 3=silicon nitride):

0=hard mask layer (e.g., Silicon Nitride)
1=dye (e.g., Si)
2=dielectric insulator (e.g., Silicon oxide)
3=thinnable membrane material (Silicon nitride)

Figure 35

(thinned silicon nitride):

Figure 36

(nanopore/s in thinned silicon nitride):

Figure 37

## (layer 3=silicon nitride, 4=other material):

0=hard mask layer (e.g., Silicon Nitride)
1=dye (e.g., Si)
2=dielectric insulator (e.g., Silicon oxide)
3=thinnable membrane material (Silicon nitride)
4=other membrane material (e.g., Hafnium oxide, all others)

Figure 38

# (free-standing layer of 4):

Figure 39

# (nanopore/s in free-standing layer of 4):

Figure 40

(lower electron absorbance in thin membrane)

Zoom series of a 7 nm diameter pore drilled in a 6 nm thick membrane (35 sec etch, square pattern)

## REFERENCES CITED IN THE DESCRIPTION

**Patent documents cited in the description**

- US 20100142259 A

- US 20100009134 A

**Non-patent literature cited in the description**

- **WANUNU et al.** *Biophys. J.,* August 2008, vol. 95
- **WANUNU et al.** *Nature Nanotechnology,* November 2010, vol. 5

- **STORM et al.** *Nature Materials,* August 2003, vol. 2